# EUROPEAN PATENT SPECIFICATION

(11) **EP 3 659 440 B1**
(45) Date of publication and mention of the grant of the patent: **18.01.2023**
(21) Application number: 18382861.5
(22) Date of filing: 28.11.2018
(51) Int. Cl.: A01N 63/22, A01P 21/00, C12R 1/07, C12N 1/20

(54) **PLANT GROWTH PROMOTING MICROORGANISM AND ENZYMES FOR SOIL BIOGENIC CYCLES**
PFLANZENWACHSTUMSFÖRDERNDER MIKROORGANISMUS UND ENZYME FÜR BIOGENE BODENZYKLEN
MICRO-ORGANISME FAVORISANT LA CROISSANCE VÉGÉTALE ET ENZYMES POUR CYCLES BIOGÉNIQUES DANS LE SOL

(43) Date of publication of application: 03.06.2020
(73) Proprietor: Bio-Iliberis Research and Development S.L., 18210 Peligros, Granada (ES)
(72) Inventor: Roca Hernández, Amalia, E-18210 Peligros, Granada (ES); Pizzarro Tobías, Paloma, E-18210 Peligros, Granada (ES)
(74) Representative: ABG Intellectual Property Law, S.L.

(56) References cited:
- CN-A- 106 434 445
- CN-A- 106 995 791
- CN-A- 107 384 840
- S Sivasakthi ET AL: "Production of Plant Growth Promoting Substance by Pseudomonas fluorescens and Bacillus subtilis Isolates from Paddy Rhizosphere Soil of Cuddalore District, Tamil Nadu, India", International Journal of Microbiological Research, 1 January 2013 (2013-01-01), pages 227-233, XP055569239, DOI: 10.5829/idosi.ijmr.2013.4.3.75171 Retrieved from the Internet: URL:https://pdfs.semanticscholar.org/8583/ 4c64b7605677c9b2329d7b52faa2aa3a1c21.pdf

## Description

### FIELD OF THE INVENTION

The invention relates to the field of microbiological strains having the capacity to promote plant resistance to at least one phytopathogen, to produce at least one plant growth-stimulating compound, and/or to solubilise insoluble phosphate and/or iron, and their applications. Specifically, the invention relates to the microorganism of the *Bacillus* sp. BIRD-6 strain with accession number CECT 9748, and supernatants of the culture.

### BACKGROUND TO THE INVENTION

In recent years, there has been an increase in awareness of the deleterious effects that inorganic chemical fertilisers and phytosanitary products exert on the environment. Depletion of soil nutrients through leaching into the waterways and causing contamination are some of the negative effects of these inorganic chemical fertilisers. Chemical phytosanitary compounds often have such an ample range of actions that they impact both the flora and small animals in the ecosystem, and may also have deleterious effects on the health of invertebrates, higher plants and animals and humans. The adverse environmental effects have brought about stricter legislations preventing their use which, together with a growing interest in ecological agriculture, has prompted the need for suitable alternatives.

The idea of eliminating the use of fertilisers and chemical environmentally-unsafe phytosanitary products is slowly becoming a reality due to the emergence of microorganisms that can serve the same purpose or even do better. This brings the idea of using microbes that can be developed for use as biological fertilisers (biofertilisers) while at the same time preventing phytopathogens. They are environmentally friendly as they are natural living organisms. They increase crop yield and production and, in addition, in developing countries, they are less expensive compared to chemical fertilisers and phytosanitaries. These biofertilizers/phytosanitaries are typically called "biocontrol microbes". These microbes can efficiently colonise the rhizosphere of plants and produce nutrients for the host plant while positively influencing the growth and development of the roots and overall plant growth through pathogen control (Vessey J.K., 2003, Plant and Soil 255: 571-86) and, in some cases, they can effectively colonize the aerial part of the plant or at least survive on them to exert their role.

*Bacillus* strains that provide improved plant growth and/or resistance to plant pathogens are well known, such as disclosed in CN107384840A, CN106434445A and CN106995791A. Furthermore, S. Sivasakthi et al teaches in the International Journal of Microbiological Research, 2013, pages 227-233 that *Bacillus* is a very abundant genus and their PGPR activity is common.

The objective of worldwide sustainable agriculture is much more likely to be achieved through the widespread use of biofertilisers/biopesticides. However, to accomplish this objective it is essential that the microorganisms display additional traits so that they can substitute other chemically synthesised products that are currently used to protect plant crops against phytopathogens and situations of abiotic stress, and as phosphate and iron supplements.

It would be particularly advantageous to have microorganisms exhibiting one or more of those additional traits to use as biofertilisers. It has proven difficult to obtain microorganisms having these characteristics and which comply with the rigorous regulations with regards to efficacy and safety.

Therefore, there is still a need in the art to provide microorganisms with improved properties to use as biofertilisers.

### SUMMARY OF ASPECTS OF THE INVENTION

The present inventors have selected a strain of the *Bacillus* spp., i.e. *Bacillus sp.* BIRD-6 strain with accession number CECT 9748, from soil sample obtained in Pinos Genii (Granada, Spain), which is capable of producing plant growth-promoting compounds (Examples 1 to 6 ) and protecting plants against environmental stress (Example 27) and phytopathogens (Examples 21 to 24). This strain is also able to solubilise insoluble phosphate and iron from soil and use the solubilised forms as nutrients (Examples 7 to 13). Moreover, this microorganism produces proteases that favour the nitrogen cycle (Example 1). Therefore, the use of microorganisms of the CECT 9748 *Bacillus sp.* BIRD-6 strain contributes to the decrease in the use of phytosanitary compounds (i.e. fungicides, etc.) and also contributes to diminish the use of nitrogenated compounds with phosphates. As shown in the Examples, said microorganisms excel in promoting plant growth in a number of plant crops of agricultural interest, and in protecting said plant crops against phytopathogens and abiotic stress.

Thus, in a first aspect, the present invention relates to a microorganism of the *Bacillus* sp. BIRD-6 strain with accession number CECT 9748.

In a second aspect, the present invention relates to a supernatant of a culture of the microorganism according to the first aspect of the invention.

In a third aspect, the present invention relates to a fertiliser, seed, root ball, substrate, active solid support comprising at least one microorganism according to the first aspect of the invention, or a supernatant according to the second aspect of the invention.

In a fourth aspect, the present invention relates to a method for stimulating plant growth, comprising a step of applying an effective amount of at least one microorganism according to the first aspect of the invention, or a supernatant according to the second aspect of the invention, or a fertiliser, substrate, active solid support or according to the third aspect of the invention, to the plant or, alternatively, a step of sowing a seed or a root ball according to the third aspect of the invention.

In a fifth aspect, the present invention relates to a method for protecting a plant against phytopathogens, comprising a step of applying an effective amount of a microorganism according to the first aspect of the invention, or a supernatant according to the second aspect of the invention, or a fertiliser, substrate, or active solid support according to the third aspect of the invention, to the plant or, alternatively, a step of sowing a seed or a root ball according to the third aspect of the invention.

In a sixth aspect, the present invention relates to a method for protecting a plant against environmental stress and phytopathogens, comprising the step of applying an effective amount of at least a microorganism according to the first aspect of the invention, or a supernatant according to the second aspect of the invention, or a fertiliser, substrate, or active solid support according to the third aspect of the invention, to the plant or, alternatively, the step of sowing a seed or a root ball according to the third aspect of the invention.

In a seventh aspect, the present invention relates to a method for solubilising an insoluble phosphate and/or insoluble iron in a solid substrate or field, comprising the step of applying at least one microorganism according to the first aspect of the invention, or a fertiliser, substrate, or active solid support according to the third aspect of the invention, to the solid substrate or field or, alternatively, the step of sowing a seed or a root ball according to the third aspect of the invention on the solid substrate or field.

In an eighth aspect, the present invention relates to the use of the microorganism according to any of the first aspect of the invention, or the supernatant according to the first aspect of the invention, or the fertiliser seed, root ball, active solid support, or soil according to the third aspect of the invention, for stimulating plant growth and/or protecting a plant against phytopathogens and/or for protecting a plant against environmental stress and/or for solubilising an insoluble phosphate and/or insoluble iron in a solid substrate or field and/or for obtaining a strain with capacity to promote resistance to growth of phytopathogens, to produce at least one plant growth-stimulating compound, and/or to solubilise insoluble phosphate and/or iron.

### DETAILED DESCRIPTION OF THE INVENTION

### 1. Microorganism of the invention

In a first aspect, the present invention relates to a microorganism of the *Bacillus sp.* BIRD-6 strain with accession number CECT 9748, hereinafter "the microorganism of the invention".

The present invention refers to a microorganism of the *Bacillus sp.* BIRD-6 strain, deposited in the Colección Española de Cultivos Tipo (CECT) with accession number CECT 9748.

The *Bacillus sp.* BIRD-6 strain is characterized in that the gene encoding the 16S RNA comprises the sequence of SEQ ID NO:3.

The processes of promoting plant resistance to at least one phytopathogen, producing at least one plant growth stimulating compound, solubilising phosphates and solubilising iron are of an independent genetic nature.

The term "capacity to promote plant resistance to at least one phytopathogen", as used herein, refers to the bacterial traits that inhibit the functioning of one or more plant pathogenic organisms. The plant resistance to at least one phytopathogen may be systemic resistance. The mechanisms by which plant resistance to at least one phytopathogen may be promoted by the microorganism of the invention include the production of produce 1-aminocyclopropane-1-carboxylate (ACC) deaminase, synthesis of one or more antibiotics, production of cell wall degrading enzymes, such as proteases, amylases, and lipases, competition, synthesis of hydrogen cyanide, induced systemic resistance, quorum quenching and production of siderophores.

In an embodiment, the phytopathogen is selected from fungi or bacteria.

In a particular embodiment, the phytopathogen is fungi. Non-limiting examples of fungi include *Fusarium oxysporum, Rhizoctonia solanii,* and*Pythium ultimum.*

In an embodiment, the capacity to promote plant resistance to at least one phytopathogen is by synthesising one or more antibiotics. Non-limiting examples of antibiotics include Tas A, sublancin, subtilosin, bacilysin, chlorotetain, subtilin, bacillaene, surfactin, iturin, and fengycin.

In an embodiment, the capacity to promote plant resistance to at least one phytopathogen is by producing cell wall degrading enzymes. The enzymes include chitinase, which degrades chitin, i.e. a residue of β-(1, 4)-N-acetyl glucosamine polymer and an integral part of the cell wall of many phytopathogenic fungi; β-1,3-glucanase, which degrades another cell wall carbohydrate; protease, which may degrade cell wall proteins; amylases, which may degrade carbohydrates and starches; and lipase, which may degrade some of the cell wall-associated lipid; all of which may to some extent individually lyse fungal cells.

In an embodiment, the capacity to promote plant resistance to at least one phytopathogen is by competing with the phytopathogen. The microorganism of the invention may outcompete the phytopathogens either for nutrients or for binding sites on the plant root. Such competition can act to limit the binding of the phytopathogen to the plant thereby making it difficult for it to proliferate.

In an embodiment, the capacity to promote plant resistance to at least one phytopathogen is by producing siderophores. Siderophores produced by plant growth promoting bacteria (PGPB) have a much higher affinity for iron than siderophores produced by either plants or fungi so that siderophores from PGPB can sequester even minute amounts of iron, thereby limiting the amount of iron that is available for phytopathogens.

The capacity of a microorganism to promote plant resistance to at least one phytopathogen may be determined by any conventional method. For example, a suitable amount of a culture of a phytopathogen, such as a fungi, may be placed onto a Potate Dextrose Agar in a Petri dish (Masago et al., 1976, Phytopathology 67:425), to which a suitable amount of a microorganism to be assayed, or of the supernatant obtained from culturing said microorganism, is applied and inhibition in the growth of the phytopathogen is monitored (Examples 21 and 24).

The capacity of a microorganism to produce cell wall degrading enzymes, such as proteases, amylases, β-glucosidades and lipases, may be evaluated by any method that is conventional in the art, such as the methods described in Examples 1, 2, 3, 4 and 5 of the present application.

The term "capacity to produce at least one plant growth stimulating compound", as used herein, refers to the property by which a strain is able to synthesise and release to the soil at least one plant growth stimulating compound. The term "plant growth stimulating compound" or "plant growth promoting compound", as used herein, refers to compounds which promote plant growth by facilitating the development of the plant root system, stem elongation, flowering, etc. Examples of plant growth stimulating compounds that may be produced by the variant strain of the invention includes, without limitation, nitrogen sources, such as amino acids; phytohormones, such as indole 3-acetic acid (IAA), gibberellic acid (GA3), cytokinin, ethylene, zeatin, and abscisic acid (ABA); enzymes that favour nitrogen assimilation or which hydrolyse proteins, such as proteases; enzymes that facilitate the carbon cycle, such as glucosidases and amylases; and plant growth regulatory compounds, such as polyamines, e.g. cadaverine (CAD).

The ability of a microorganism to produce plant growth stimulating or promoting compounds can be determined by means of any conventional method, for example, by means of an early plant growth stimulation assay. Briefly, said assay comprises sowing seeds, e.g. corn, barley, grass, or Avex III (herbage mixture), in a homogenous mixture made up of agricultural soil mixed with sand (silica) and a culture of the microorganism to be assayed (in a suitable population density) (Examples 15 to 17), preferably adhered to a substrate (Example 18), or a solid support (Example 19) or a seed (Example 20). The system is incubated under conditions that allow seed germination (e.g. at 20 °C in the dark). The percentage of seed germination and/or the length of the stem is determined a week after sowing. The ability of a microorganism to produce plant growth stimulating or promoting compounds can also be determined by supplementing seeds or root balls prior to sowing (Example 20).

The ability of a microorganism to produce each type of plant growth stimulating compound may also be determined separately. Production of phytohormones may be determined by suitable chromatography techniques, such as reverse phase chromatography and gas chromatography-mass spectrometry with selective ion monitoring (GC-MS-SIM), as described by Perrig et al., 2007, Appl Microbiol Biotechnol 75:1143-50). Ethylene production may be determined by, for example, gas chromatography-flame ionisation detection. Production of IAA may be assessed using amended LB (Naik *et al*., 2008; as above) and placing a Whatman paper disk with the inoculated microorganism over the medium. After incubation, Whatman paper disk is reacted with Salkowsky reactive. If a reddish colour appeared in the Whatman paper after 1 h of incubation, the production of IAA is considered positive (Examples 1 and 6). One skilled in the art will understand that other assays that serve to evaluate the plant growth stimulation or promotion can be conducted, such as, for example, the assays mentioned in international patent application WO 2011/147826.

In an embodiment, the at least one plant growth-stimulating compound is a phytohormone. In a preferred embodiment, the phytohormone is IAA.

The ability of a microorganism to hydrolyse proteins refers to the bacterial capacity to produce proteases that are able to hydrolyse or break down the proteins and peptides that are present in the soil. The hydrolysis of proteins in soil favours nitrogen uptake in plants as it liberates small peptides and amino acids that favour plant nutrition. Many kinds of proteases that are secreted are known as endopeptidases, which are characterized by their preferential action at the peptide bonds in the inner regions of the polypeptide chain away from the N and C termini (Rao et al., 1998, Microbiol Mol Biol Rev 62:597-635). One of the four subgroups of endoproteases are the serine proteases that include the subtilisins, which are endoproteases produced by *Bacillus* sp. strains (Rani et al., 2012 Int J Curr Life Sci 2:12-8).

The capacity of a microorganism to hydrolyse proteins may be determined by any conventional method. For example, a microorganism to be assayed may be cultured in soil medium containing skimmed milk (Naik et al., 2008, Microbial Ecology 56:492-504). Protein hydrolysis is detected when a clear halo forms in the media surrounding a given colony (Examples 1 and 2).

The proteins may be proteins found in soil or soil proteins.

In another embodiment, the at least one plant growth-stimulating compound is a protease.

The ability of a microorganism to produce enzymes that facilitate the carbon cycle, such as glucosidases and amylases, may be evaluated by any method that is conventional in the art, such as the methods described in Examples 1, 3 and 4 of the present application.

In another embodiment, the at least one plant growth-stimulating compound is a glucosidase. In another preferred embodiment, the glucosidase is a β-glucosidase.

In another embodiment, the at least one plant growth-stimulating compound is an amylase.

The term "capacity to solubilise phosphate", as used herein, refers to the ability of a strain is able to release and solubilise phosphate from an insoluble phosphate source, such as the phosphate present, for example, in soils or in rock phosphate (e.g. dibasic calcium phosphate (CaHPO₄ ×2H₂O), tribasic calcium phosphate (Ca₅(PO₄)₃OH or TPC), etc.), and using it as a phosphorus source. Phosphate solubilisation by a microorganism may be assessed by methods that are conventional in the art. For example, by inoculating a culture of said microorganism in a medium containing one or more insoluble phosphates as the sole phosphorus source (e.g., tricalcium phosphate, etc.), incubating under suitable conditions and counting the viable microorganisms; under these conditions, the maintenance or an increase of the bacterial population density is indicative that said microorganisms solubilize said insoluble phosphate/phosphates and use it/them as a phosphorus source (Rodriguez & Fraga, 1999, Biotechnology Advances 17:319-39). Another assay which allows clarifying if a microorganism is capable of solubilising insoluble phosphates comprises seeding them in solid media specialised for this purpose, such as Agar Pikovskaya (Naik *et al.,* 2008; as above), in which the microorganism generates a microenvironment due to the production of organic acids, which translates into a visible halo in the medium (Examples 1, 7 and 8). Another example consists of adding a microorganism to be assayed to water containing molten phosphoric rock, in the presence of a carbon source and with aeration, and monitoring phosphorous solubilisation (Examples 10, 11 and 12).

The term "capacity to solubilise iron", as used herein, refers to the ability of a strain to solubilise insoluble iron present, for example, in soils or in rock phosphate (e.g. iron oxides, etc.). The most common way for the microorganisms to solubilize iron is by means of producing, which are small, high affinity iron chelating compounds that are taken up by the strain of the invention and facilitate iron uptake rate by plants. Siderophore production may be determined by any conventional method, for example, by inoculating pure bacterial culture in plates containing CAS agar (Alexander & Zuberer, 1991, Biology Fertility Soils 12:39) and observing for orange colour formation around each colony upon incubation (Examples 1 and 9). The ability of a microorganism to solubilise iron can also be determined by inoculating a culture of said microorganism in a medium containing insoluble iron as the only iron source (e.g., ferric trichloride, etc.), and counting the viable microorganisms, as described in WO 2010/018210. Under these conditions, the maintenance of or an increase in the bacterial population density is indicative that said microorganisms solubilise said insoluble iron and use it/them as an iron source (Rodriguez & Fraga,1999, as above). Another example consists in inoculating a culture of said microorganism in a suitable culture medium containing no iron and adding molded phosphoric rock containing 15 to 30% de P₂O₅, incubating under suitable conditions and counting the viable microorganisms; under these conditions, the maintenance or an increase of the bacterial population density is indicative that said microorganisms use iron, or, the formation of a greenish-yellow colour in the supernatants of the cultures is indicative of the excretion of certain siderophores into the culture medium (Example 13).

The CECT 9748 *Bacillus* sp. BIRD-6 strain promotes plant resistance to at least one phytopathogen, produces at least one plant growth-stimulating compound, hydrolyses proteins and sugars derived from vegetable matter, solubilises insoluble phosphate, and solubilises insoluble iron, as demonstrated in the Examples of the present application.

Additionally, the present inventors have demonstrated that the CECT 9748 *Bacillus* sp. BIRD-6 strain has also been shown to be innocuous to other plant growth-promoting microorganisms (Example 30) and heterotrophic soil microorganisms (Examples 30 and 31).

It will be recognised that the supernatant of the invention contains the products derived from the metabolism of the microorganism of the invention, i.e. compounds which promote plant resistance to at least one phytopathogen, and/or at least one plant growth-stimulating compound, and/or compounds which hydrolyse proteins and sugars derived from vegetable matter, and/or any compound which helps solubilising insoluble phosphate and/or iron. These features allow the supernatant of the invention to have multiple applications in the field of agriculture, such as use as a fertiliser and plant strengthener.

Therefore, the invention also contemplates the supernatant of a culture of the microorganism according to the invention, hereinafter "the supernatant of the invention".

As used herein, the term "supernatant" refers to a cell-free culture medium which has been conditioned by the growth of a microorganism and which, as a result of the conditioning, contains compounds that have been synthesised and secreted by the microorganism. In order to obtain a supernatant according to the invention, a culture medium is inoculated with the strain of the invention, which is cultured under suitable conditions. For example, the strain of the invention may be cultured in a minimal culture medium comprising a carbon source that can be used by the strain. Said carbon source may comprise, without limitation, glucose, sucrose, lactose, benzoic acid, citric acid, or combinations thereof. Virtually any culture medium suitable for the development and growth of the microorganism of the invention can be used in putting said method into practice. Additionally, the culture medium may comprise other media specific for achieving production of the desired metabolite. Said culture media are well known in the state of the art and preparing them is routine practice for one skilled in the art. The strain may be cultured at a temperature between 15°C and 40°C until reaching a cell density equal to or greater than 10⁹ cfu/mL. The cells are then removed from the medium by any suitable means, such as decantation, centrifugation or filtration.

Where the culture broth or supernatant is not going to be used immediately, it can be preserved until use under cold conditions, e.g. at 4°C, -20°C, or -80°C.

A biologically pure culture of the microorganism of the invention is an additional aspect of the invention.

### 2. Products containing the strain of the invention

### 2.1. Fertiliser

In another aspect, the invention relates to a fertiliser comprising the microorganism of the invention, hereinafter "the first fertiliser of the invention".

The term "microorganism of the invention" has been described previously in detail and its definition and embodiments are included here by reference.

The term "fertiliser", as used herein, refers to any compound which is added to a soil to facilitate plant growth. The strain of the invention can be added to virtually any fertiliser, either solid or liquid, for putting the present invention into practice. Non-limiting illustrative examples of fertilisers: single nutrient fertilisers, binary fertilisers, and NPK-type fertilisers, and fertilisers based on a mixture of microelements. The main nitrogen-based single nutrient fertiliser is ammonia or its solutions. Ammonium nitrate and urea are also widely used. The main straight phosphate fertilisers are the superphosphates, which comprise varying amounts of various phosphates, such as P₂O₅. Binary fertilisers, or NP, NK or PK fertilisers, provide two-component combinations of nitrogen, phosphorus and potassium. NPK fertilisers are three-component fertilisers providing nitrogen, phosphorus, and potassium.

Any liquid fertiliser with a pH comprised between 2 and 10 may also be used for putting this invention into practice. Non-limiting examples of liquid fertilisers include fertilisers based on fulvic acids, liquid fertilisers with different compositions, such as 16:2:4 mixtures, 20:5:0 mixtures, etc., or various types of fertilisers in drops with an NPK composition of 8:9:9, 16:4:4, etc. The microorganism of the invention can be added to the fertiliser.

The fertiliser herein described may contain, if desired, other ingredients or constituents commonly used in agricultural compositions, such as surfactants, adhesives, active agents, or pH regulators, provided that they do not jeopardize or compromise the viability of the microorganism of the invention. Said ingredients or constituents commonly used in agricultural compositions are generally known to those skilled in the art. The main micronutrients widely used are molybdenum, zinc, and copper although the micronutrient needs depend on the plant. For example, sugar beets appear to require boron, and legumes require cobalt.

The fertiliser of the invention can be obtained by conventional methods by mixing said fertiliser with a culture containing the microorganism the invention. Said mixture is made with the suitable weight:volume (w:v) proportion which is greatly variable depending on, among other factors, the colony forming units (cfu) present in the culture and the cfu per unit of measure (g or mL) of fertiliser to be obtained in the supplemented fertiliser of the invention.

In a particular embodiment, between 0.01 and 1 mL of culture containing 10⁷ cfu of the microorganism of the invention are mixed with 1 g of solid fertiliser. In another specific embodiment, 1 mL of culture containing 10⁷ cfu of the microorganism of the invention is mixed with 1 mL of liquid fertiliser with a pH comprised between 2 and 10. It will be understood that, when more than one microorganism of the invention is added to the fertiliser, the previous ratios are calculated using the combined cultures.

In order to facilitate adhesion of the microorganism of the invention to the fertiliser, a suitable adhesive may be used. Virtually any agriculturally acceptable adhesive may be used. Non-limiting examples of said adhesives include acacia gum and polymers based on alginate, such as calcium alginate. The amount of adhesive may vary but it is typically equal to or less than 1% by weight of the total weight of the fertiliser.

The fertiliser of the invention may be obtained by mixing a fertiliser with a pure culture containing the CECT 9748 *Bacillus* sp. BIRD-6 strain, or with one or more than one pure cultures each containing a different microorganism of the invention. Conveniently, the fertiliser adsorbs at least 10³ cfu of the microorganism of the invention per gram of fertiliser, at least 10⁴ cfu/g of fertiliser, at least 10⁵ cfu per g of fertiliser, at least 10⁶ cfu/g of fertiliser, at least 10⁷ cfu/g of fertiliser, at least 10⁸ cfu/g of fertiliser, at least 10⁹ cfu/g of fertiliser, at least 10¹⁰ cfu/g of fertiliser, or more.

The mixture of the fertiliser with the culture containing the microorganism of the invention can generally be made based on a weight:volume (w:v) proportion that varies greatly, depending, among other factors, on the cfu/mL present in the culture and on the cfu per gram of fertiliser to be obtained in the fertiliser of the invention.

The fertiliser of the invention may comprise only the microorganism of the invention or also different microorganisms of interest in agriculture, e.g. *Pseudomonas, Azotobacter,* other *Bacillus* strains, et cetera.

The presence of the microorganism of the invention in the fertiliser provides the fertiliser would contribute not only to reducing the environmental problem relating to the accumulation of insoluble phosphate on arable soils since it would enable its assimilation, but it would also contribute to reducing pathogen growth on the treated surface since it would remove iron, a necessary element for pathogens; from the surrounding area. Fertilisers thus supplied would play a dual role in the development of plants, namely (i) facilitating nutrients and stimulating growth and (ii) preventing pathogen growth (e.g., fungi, pathogenic bacteria, etc.) for plants. When the fertiliser of the invention is applied in the aerial part of the plant it prevents the proliferation of phytopathogens in the phyllosphere.

It will be understood that the supernatant of the invention also provides beneficial compounds to a fertiliser. Thus, in another aspect, the invention relates to a fertiliser comprising at least one supernatant of the invention, hereinafter "the second fertiliser of the invention".

The term "supernatant of the invention" has been described previously in detail and their definitions and embodiments are included here by reference.

The particular features of the first fertiliser of the invention may be conveniently adapted for the second fertiliser of the invention.

### 2.2. Substrate

As one skilled in the art will appreciate, the application of the microorganism of the invention to plants may be facilitated by fixing the microorganism on a substrate. The substrate will advantageously favour the proliferation of said strain in the plant rhizosphere. Therefore, in another aspect, the invention relates to a substrate comprising the microorganism of the invention, hereinafter "the first substrate of the invention".

The term "microorganism of the invention" has been described previously in detail and its definition and embodiments are included here by reference.

The term "substrate", as used herein, is an inert soilless substrate that is composed mainly of organic elements and that is able to sustain plants. Virtually any substrate that meets said conditions may be used in the present invention. Non-limiting examples of substrates include peat (peat moss), bark, coir dust, cork dust, cellulose, previously dried and ground algae extracts, compost, and mixtures thereof.

The substrate of the invention may be obtained by mixing said substrate with a pure culture containing the microorganism of the invention, or with more than one pure culture each containing a different microorganism according to the invention. Conveniently, the substrate adsorbs at least 10³ cfu of the microorganism of the invention per gram of substrate, at least 10⁴ cfu/g of substrate, at least 10⁵ cfu per g of substrate, at least 10⁶ cfu/g of substrate, at least 10⁷ cfu/g of substrate, at least 10⁸ cfu/g of substrate, at least 10⁹ cfu/g of substrate, at least 10¹⁰ cfu/g of substrate, or more.

The mixture of the substrate with the culture containing the microorganism of the invention can generally be made based on a weight:volume (w:v) proportion that varies greatly, depending, among other factors, on the cfu/mL present in the culture and on the cfu per gram of substrate to be obtained in the substrate of the invention. Nevertheless in a particular embodiment, said ratio substrate:culture containing the microorganism of the invention is comprised between 1:0.01 and 1:1 (w:v); in a specific embodiment, 1 ml of culture containing 10⁷of the microorganism of the invention is mixed with 1 gram of substrate.

The substrate of the invention may additionally comprise different microorganisms of interest in agriculture. The microorganisms may be combined since the microorganism of the invention does not exert detrimental effects on a range of heterotrophic bacteria or bacteria of interest in agriculture (Examples 30 and 31).

It will be understood that the supernatant of the invention also provides beneficial compounds to a substrate. Thus, in another aspect, the invention relates to a substrate comprising at least one supernatant of the invention, hereinafter "the second substrate of the invention".

The term "supernatant of the invention" has been described previously in detail and their definitions and embodiments are included here by reference.

The particular features of the first substrate of the invention may be conveniently adapted for the second substrate of the invention.

### 2.3. Active solid support

As one skilled in the art will understand, the application of the strain of the invention to plants may be facilitated by fixing the strain on a solid support. The solid support will advantageously favour the proliferation of said strain in the plant rhizosphere. Therefore, in another aspect, the invention relates to a solid support comprising the microorganism of the invention, hereinafter "the first active solid support of the invention".

The term "microorganism of the invention" has been described previously in detail and its definition and embodiments are included here by reference.

The term "solid support" or "active solid support", as used herein, is an inert solid support which has a high surface area to enable a high adsorption capacity of microorganisms (Busscher & Weerkamp, 1999, FEMS Microbiology Letters 46:465).

Virtually any solid support that meets said conditions may be used in the present invention. Non-limiting examples of solid supports include clay, such as bentonite, montmorillonite, talc, sepiolite and zeolite, perlite, styrofoam, vermiculite, talcum, rockwool, and mixtures thereof.

The active solid support of the invention may be obtained by mixing said solid support with a pure culture containing the microorganism of the invention, or with more than one pure culture each containing a different microorganism of the invention. Conveniently, the solid support adsorbs at least 10³ cfu of the microorganism of the invention per gram of solid support, at least 10⁴ cfu/g of solid support, at least 10⁵ cfu per g of solid support, at least 10⁶ cfu/g of solid support, at least 10⁷ cfu/g of solid support, at least 10⁸ cfu/g of solid support, at least 10⁹ cfu/g of solid support, at least 10¹⁰ cfu/g of solid support, or more.

The mixture of the active solid support with the culture containing the microorganism of the invention can generally be made based on a weight:volume (w:v) proportion that varies greatly, depending, among other factors, on the cfu/mL present in the culture and on the cfu per gram of solid support to be obtained in the active solid support of the invention. Nevertheless in a particular embodiment, said ratio solid support:culture containing the microorganism of the invention is comprised between 1:0.01 and 1:1 (w:v); in a specific embodiment, 1 gram of solid support is mixed with 1 ml of culture containing 10⁷of the strain of the invention.

The active solid support of the invention may additionally comprise different microorganisms of interest in agriculture.

It will be understood that the supernatant of the invention also provides beneficial compounds to a solid support. Thus, in another aspect, the invention relates to a solid support comprising at least one supernatant of the invention, hereinafter "the second active solid support of the invention".

The term "supernatant of the invention" has been described previously in detail and their definitions and embodiments are included here by reference.

The particular features of the first active solid support of the invention may be conveniently adapted for the second active solid support of the invention.

### 2.4. Seed

In another aspect, the present invention relates to a seed comprising the microorganism of the invention, hereinafter "the seed of the invention".

The term "microorganism of the invention" has been described previously in detail and its definition and embodiments are included here by reference.

The term "seed", as used herein, refers to an embryonic plant enclosed in a protective outer covering, which is formed as part of the process of reproduction in plants. The seed may be the seed of any plant with agricultural, forestry, food (for human or animal purposes), ornamental, or energy interest, etc.

The microorganism of the invention may be present partially or completely on the seed surface.

The seed of the invention may be obtained by conventional methods. By way of illustration, it can be obtained by submerging the seed in a suspension or pure culture of the microorganism of the invention or, alternatively, by spraying said suspension or culture on the seeds. The suspension or pure culture may contain more than one microorganism of the invention. Other methods include mixing the seed and the microorganism of the invention previously incorporated in a solid support to facilitate adhesion of the microorganism of the invention to said seed.

The seed of the invention may comprise at least 10³ cfu of the microorganism of the invention per gram of seed, at least 10⁴ cfu/g of seed, at least 10⁵ cfu per g of seed, at least 10⁶ cfu/g of seed, at least 10⁷ cfu/g of seed, at least 10⁸ cfu/g of seed, at least 10⁹ cfu/g of seed, at least 10¹⁰ cfu/g of seed, or more.

Furthermore, in order to improve adhesion of the microorganism of the invention to the seed, an agricultural type adhesive containing a suitable concentration of said strain, such as an organic type gel or polymer, such as acacia gum or alginate polymers or other polymers such as CARBOPOL^{®} GEL, all of which are inert for the microorganism of the invention, may be used.

The seed of the invention may additionally comprise different microorganisms of interest in agriculture.

It will be understood that the supernatant of the invention also provides beneficial compounds to a seed. Thus, in another aspect, the invention relates to a seed comprising at least one supernatant of the invention, hereinafter "the second seed of the invention".

The term "supernatant of the invention" has been described previously in detail and their definitions and embodiments are included here by reference.

The particular features of the first seed of the invention may be conveniently adapted for the second seed of the invention.

### 2.5. Root ball

In another aspect, the present invention relates to a root ball comprising the microorganism of the invention, hereinafter "the first root ball of the invention".

The term "microorganism of the invention" has been described previously in detail and its definition and embodiments are included here by reference.

As used herein, the term "root ball" refers to the main mass of roots at the base of a plant. The root ball is of particular importance when a plant is re-potted or planted out in the ground since the quality and preparation of the root ball will determine how well the plant will survive this transplantation. The root ball may be the root ball of with agricultural, forestry, food (for human or animal purposes), ornamental, or energy interest, etc. The microorganism of the invention may completely or partially coat the root ball.

The root ball of the invention may be obtained by conventional methods. By way of illustration, it can be obtained by submerging the seed in a suspension or pure culture of the microorganism of the invention or, alternatively, by spraying said suspension or culture on the root ball. The suspension or pure culture may contain more than one microorganism of the invention. Other methods include mixing the root ball and the microorganism of the invention previously incorporated in a solid support to facilitate adhesion of the microorganism of the invention to said root ball.

The root ball of the invention may comprise at least 10³ cfu of the microorganism of the invention per gram of root ball, at least 10⁴ cfu/g of root ball, at least 10⁵ cfu per g of root ball, at least 10⁶ cfu/g of root ball, at least 10⁷ cfu/g of root ball, at least 10⁸ cfu/g of root ball, at least 10⁹ cfu/g of root ball, at least 10¹⁰ cfu/g of root ball, or more.

Furthermore, to improve adhesion of the microorganism of the invention to the root ball, an agricultural type adhesive containing a suitable concentration of said strain, such as an organic type gel or polymer, such as acacia gum or alginate polymers, all of which are inert for the microorganism of the invention, may be used.

The root ball of the invention may additionally comprise different microorganisms of interest in agriculture.

It will be understood that the supernatant of the invention also provides beneficial compounds to a root ball. Thus, in another aspect, the invention relates to a root ball comprising at least one supernatant of the invention, hereinafter "the second root ball of the invention".

The term "supernatant of the invention" has been described previously in detail and their definitions and embodiments are included here by reference.

The particular features of the first root ball of the invention may be conveniently adapted for the second root ball of the invention.

### 3. Methods of use

It will be readily appreciated that the microorganism of the invention has multitude of applications in agriculture. The methods that exploit the use of the microorganism of the invention for stimulating plant growth, for protecting a plant against pathogens, for protecting a plant against environmental stress and/or for solubilizing an insoluble phosphate and/or insoluble iron in a solid substrate or soil are also part of the present invention.

### 3.1. Method for stimulating plant growth

The presence of the microorganism of the invention or the supernatant of the invention is particularly advantageous in agriculture because they promote the simultaneous germination of the seeds, provide for homogenous growth of the plants of the same crop, and coordinate the flowering, fruiting, and fruit maturation periods.

In another aspect, the invention relates to a method for stimulating plant growth, hereinafter "the method for stimulating plant growth of the invention", comprising the step of applying an effective amount of the microorganism of the invention.

The capacity of the microorganism of the invention to produce at least one plant growth stimulating compound and/or to solubilise insoluble phosphate and/or iron is particularly advantageous

Alternatively, the method for stimulating plant growth of the invention may comprise the step of applying the supernatant of the invention to the plant.

It will be readily appreciated that the microorganism of the invention can be applied in different ways, i.e. directly to the soil, preferably to the radicular area of influence of the plant or the phyllosphere, or as part of a fertiliser, or adhered to a substrate or an active solid support.

Alternatively, the method for stimulating plant growth of the invention may comprise the step of applying the fertiliser of the invention to the plant.

Alternatively, the method for stimulating plant growth of the invention may comprise the step of applying the substrate of the invention to the plant.

Alternatively, the method for stimulating plant growth of the invention may comprise the step of applying the active solid support of the invention to the plant.

Alternatively, the method for stimulating plant growth of the invention may comprise the step of applying the soil of the invention to the plant.

Alternatively, the growth of a plant may be stimulated by means of sowing a seed or a root ball of said plant supplemented with the microorganism of the invention, i.e. the seed or root ball of the invention. In this case, the microorganism of the invention will replicate and populate the area of soil surrounding the seed or root, and exert its function in stimulating plant growth.

Alternatively, the method for stimulating plant growth of the invention may comprise the step of sowing the seed of the invention or the root ball of the invention near the plant whose growth is to be stimulated. Provided that the seed or root ball are planted sufficiently near the radicular area of influence of the plant whose, the microorganism of the invention will replicate and populate nearby areas of rhizosphere.

The terms "supernatant of the invention", "fertiliser of the invention", "substrate of the invention", "active solid support of the invention", "seed of the invention", and "root ball of the invention", have been described previously in detail and their definitions and embodiments are included here by reference.

The term "effective amount", as used in the context of this aspect of the invention, refers to the amount of the microorganism of the invention, the supernatant of the invention, or the products containing said microorganism or supernatant according to the invention, that is required to stimulate, enhance or induce plant growth, i.e. to increase the biomass of said plant. The term "plant biomass", as used herein, refers to the amount of organic material contained in a plant, i.e., the organic material constituting both the aerial part of the plant, that is, the stem, the trunk, the leaves, the branches, the fruit, the inflorescences, etc. (aerial biomass), and the underground part thereof, i.e., the roots, calluses, tubercles, etc. (underground biomass). The "plant biomass" is often measured as the dry mass or weight (or "fresh weight," where appropriate) of the plant. As known the skilled person, there are multiple methods and parameters serving to calculate plant growth or the increase in biomass in the state of the art, including, without limitation, the growth rate, the relative growth rate, the leaf area ratio, the specific leaf area, the leaf proportion, the net assimilation rate, the stem proportion, the root proportion, and the dry matter content.

The effective amount of a microorganism according to this aspect of the invention may be determined by methods that are conventional in the art. For example, different amounts of the microorganism of the invention, the supernatant of the invention, or the products containing said microorganism or supernatant according to the invention, may be applied to different plants which are at the same stage of development. Plant growth, i.e. the increase in biomass, is monitored over time to determine which of the tested amounts is effective. The increase in plant biomass may be measured as a % of biomass increase when compared with a plant that has not been treated with the microorganism of the invention.

Other indicators of plant growth stimulation may include increased in stem length, increased root length, increased number of secondary roots, earlier germination of seeds, number of germinating seeds, earlier flowering, amount and quality of the fruits, and earlier harvests.

The effective amount will depend on the particular plant, growth stage and the radicular area of influence, which may be translated as the size of the plant. The effective amount may be measured in total cfu, cfu per gram of rhizospheric soil or any other convenient unit. The effective amount of the microorganism of the invention may be between 10³ and 10⁹ cfu of the microorganism or the invention per gram of rhizospheric soil. The effective amount of the supernatant of the invention may be between 1 mL and 10 L per m² of soil surface. The effective amount of the fertiliser, substrate, or active solid support of the invention may be an amount which results in between 10³ and 10⁹ cfu of the microorganism or the invention per gram of rhizospheric soil. Said amounts may be applied in a single administration or in several administrations.

The growth of virtually any plant may be stimulated according to this method. By way of illustration, any plant having agricultural interest may be used for this method, including plants of interest in human or animal food, plants of ornamental interest, plants of forestry interest, plants of energy interest, and so on. Non-limiting examples include oat, barley, corn, wheat, tomato, pepper, cucumber, eggplant, grass, sorghum, rose bushes, geraniums, daisies, soya, any tree, such as fruit trees, oak tree, walnut tree, beech tree, pine tree, etc.

The method for stimulating growth of the invention may be particularly effective in the early stages of growth.

In an embodiment, the stimulation of plant growth is caused by a stimulation of seed germination or by a stimulation of early growth.

There are various mechanisms by which the microorganisms of the invention may stimulate plant growth. First, the production of plant growth-stimulating compounds, such as phytohormones, may also play a role. For example, rhizobacterial IAA changes plant auxin pools, ultimately increasing root length and surface area, and in the process increasing the level of root exudates available for uptake by plants; cytokinin promotes cell division, or cytokinesis, in plant roots and shoots; gibberellins regulate various developmental processes, including stem elongation, germination, dormancy, flowering, flower development and leaf and fruit senesc ence.

Second, a large proportion of organic nitrogen present in soils is in the form of peptides and proteins. By producing proteases, the soil peptides and proteins are hydrolysed thereby making the N available for plants to uptake.

Third, the microorganism of the invention has been shown to form biofilms on the surface of roots of horticultural plants which has a protective effect against pathogenic organisms and, in turn, promotes plant growth.

Fourth, by converting insoluble organic and inorganic phosphate and iron to forms which can be readily accessible to plants.

Fifth, the production of siderophores can prevent or lessen pathogen proliferation by reducing the amount of iron that is available to a pathogen, which in turn promotes plant growth.

In an embodiment, the seeds are from a monocotyledon. In another embodiment, the seeds are from a dicotyledon.

### 3.2. Method for protecting a plant against phytopathogens

The CECT 9748 *Bacillus sp.* BIRD-6 strain has been shown to form biofilms on the surface of plant roots (Examples 25 and 26). Such biofilms occupy the ecological niche completely, preventing the colonisation of other types of rhizosphere pathogenic organisms. Additionally, the ability of the CECT 9748 *Bacillus sp.* BIRD-6 strain to produce siderophores (Example 1) is also important for protecting plants against pathogens. Phytopathogens can be maintained at low levels by sequestering the iron that is available on the soil with the production of siderophores.

Thus, in another aspect, the present invention relates to a method for protecting a plant against phytopathogens, comprising a step of applying an effective amount of a microorganism according to the invention to the plant.

In another embodiment, an effective amount of different microorganisms of interest in agriculture are also applied to the plant.

Alternatively, the method for protecting a plant against phytopathogens may comprise a step of applying an effective amount of a supernatant according to the invention to the plant.

Alternatively, the method for protecting a plant against phytopathogens may comprise a step of applying an effective amount of a biofertiliser according to the invention to the plant.

Alternatively, the method for protecting a plant against phytopathogens may comprise a step of applying an effective amount of a substrate according to the invention to the plant.

Alternatively, the method for protecting a plant against phytopathogens may comprise a step of applying an effective amount of an active solid support according to the invention to the plant.

Alternatively, the method for protecting a plant against phytopathogens may comprise a step of sowing a seed according to the invention.

Alternatively, the method for protecting a plant against phytopathogens may comprise a step of sowing a root ball according to the invention.

The terms "microorganism of the invention", "supernatant of the invention", "fertiliser of the invention", "substrate of the invention", "active solid support of the invention", "seed of the invention", and "root ball of the invention", have been described previously in detail and their definitions and embodiments are included here by reference.

The term "phytopathogen", as used herein, refers to any organism that is pathogenic to plants. Examples of phytopathogenic organisms include, without limitation fungi; fungus-like organisms, such as oomycetes and phytomyxea; bacteria, such as *Erwinia* spp., *Burkholderia, Xanthomonas* spp., and *Pseudomonas* spp.; phytoplasmas and spiroplasmas; viruses and viroids; protozoa, such as *Phytomonas;* nematodes; and parasitic plants, such as mistletoe and dodder.

In an embodiment, the phytopathogen is a fungi. Examples of fungi whose growth is halted by the microorganism of the invention, including without limitation, *Phytoptora, Sclerotinia, Fusarium, Alternaria,* etcetera.

The term "effective amount", as used in the context of this aspect of the invention, refers to the amount of the microorganism of the invention, regardless of the form of presentation that is required to obtain the desired effect of protecting a plant against the colonisation of phytopathogens and thereby obtaining beneficial results.

The effective amount of a microorganism according to this aspect of the invention may be determined empirically by methods that are conventional in the art. For example, different amounts of the microorganism of the invention, may be applied to different plants which are at the same stage of development and suffer from the same type of pathogenic infection or infestation. Plant growth, i.e. the increase in biomass, and/or the presence of the phytopathogen is monitored over time to determine which of the tested amounts is effective.

The effective amount will depend on the particular plant, growth stage and the radicular area of influence, which may be translated as the size of the plant, and also on the particular type of phytopathogen that is colonising the plant. The effective amount may be measured in total cfu of the microorganism of the invention, cfu of the microorganism of the invention per gram of rhizospheric soil or any other convenient unit. The effective amount of the microorganism of the invention may be between 10³ and 10⁹ cfu per gram of rhizospheric soil. The effective amount of the supernatant of the invention may be between 1 mL and 10 L per m² of soil surface. The effective amount of the fertiliser, substrate, or active solid support of the invention may be an amount which results in between 10³ and 10⁹ cfu per gram of rhizospheric soil. Said amounts may be applied in a single administration or in several administrations.

The normal state of virtually any plant may be restored according to this method. By way of illustration, any plant having agricultural interest may be used for this method, including plants of interest in human or animal food, plants of ornamental interest, plants of forestry interest, plants of energy interest, and so on. Non-limiting examples include oat, barley, corn, wheat, tomato, pepper, cucumber, strawberry, blackberries, eggplant, grass, sorghum, rose bushes, geraniums, daisies, soya, any tree, such as fruit trees, oak tree, walnut tree, beech tree, pine tree, etc.

Without wishing to be bound by any theory, it has been described that biofilm formation by plant growth promoting microorganisms on the roots of plants provides them with a screen to block the proliferation of phytopathogens on the roots (Rafique et al., 2015. The Battle Against Microbial Pathogens: Basic Science, Technological Advances and Educational Programs., Chapter: Bacterial biofilm formation and its role against agricultural pathogens, pp. 373-82. Publisher: Formatex Research Center, Spain., Ed.; A. Mendez Vil). Moreover, the sequestration of insoluble iron from the soil by production of siderophores limits its availability for other phytopathogens, which in turn halts their proliferation.

### 3.3. Method for protecting a plant against environmental stress

The present inventors have demonstrated that the microorganism of the invention is be able to form biofilms both on abiotic surfaces (Examples 25 and 26) and on the surface of plant roots. Without wishing to be bound by any theory, it has been described that biofilm formation by plant growth promoting microorganisms on the roots of plants provides them with greater protection against abiotic stress situations such as, for example, adverse weather conditions, changes in temperature (e.g. high or low temperatures), high salinity, and desiccation or water stress conditions (e.g. drought, etc.) (Tyerman et al., 2012, Plant Cell & Environment 25:173-94; Mauch-Mani & Slusarenko, 1996, The Plant Cell 8:203-12, Chen, et al., (2016), Physiologia plantarum, 158(1), 34-44 and Kasim, W. A.,et al. (2016). Annals of Agricultural Sciences, 61(2), 217-227.).

In another aspect, the invention relates to a method for restoring the normal state of a plant after suffering environmental stress, hereinafter "the method for protecting a plant against environmental stress of the invention", comprising the step of applying an effective amount of the microorganism of the invention.

Alternatively, the method for protecting a plant against environmental stress of the invention may comprise the step of applying the supernatant of the invention to the plant.

It will be readily appreciated that the microorganism of the invention can be applied in different ways, i.e. directly to the soil, preferably to the radicular area of influence of the plant, or as part of a fertiliser, or adhered to a substrate or solid support.

Alternatively, the method for protecting a plant against environmental stress of the invention may comprise the step of applying the fertiliser of the invention to the plant.

Alternatively, the method for protecting a plant against environmental stress of the invention may comprise the step of applying the substrate of the invention to the plant.

Alternatively, the method for protecting a plant against environmental stress of the invention may comprise the step of applying the active solid support of the invention to the plant.

Alternatively, the protection of a plant against environmental stress may be achieved by means of adhering the microorganism of the invention to the seed or root ball of the plant to be protected. Accordingly, the method for protecting a plant against environmental stress of the invention may comprise the step of sowing the seed of the invention or the root ball of the invention. Protection may also be achieved if the seed or root ball of the invention are planted near the plant to be protected. Provided that the seed or root ball are planted sufficiently near the radicular area of influence of the plant, the microorganism of the invention will replicate and populate nearby areas of rhizosphere.

The terms "microorganism of the invention", "supernatant of the invention", "fertiliser of the invention", "substrate of the invention", "active solid support of the invention", "seed of the invention", and "root ball of the invention", have been described previously in detail and their definitions and embodiments are included here by reference.

The term "environmental stress", as used herein, refers to abiotic situations which affect the conditions that are optimal for plant growth. Non-limiting examples of environmental stress include adverse weather conditions, such as intense cold or frosts, changes in temperature, including high temperatures or thermal shock, desiccation or water stress conditions, salinity, etc.

The term "effective amount", as used in the context of this aspect of the invention, refers to the amount of the microorganism of the invention, regardless of the form of presentation that is required to obtain the desired effect of protecting a plant from an environmental stress situation and thereby obtaining beneficial results.

The effective amount of a microorganism according to this aspect of the invention may be determined empirically by methods that are conventional in the art. For example, different amounts of the microorganism of the invention, may be applied to different plants which are at the same stage of development and have suffered the same type of environmental stress. Plant growth, i.e. the increase in biomass, is monitored over time to determine which of the tested amounts is effective.

The effective amount will depend on the particular plant, growth stage and the radicular area of influence, which may be translated as the size of the plant, and also on the particular type of environmental stress suffered by the plant. The effective amount may be measured in total cfu of the microorganism of the invention, cfu of the microorganism of the invention per gram of rhizospheric soil or any other convenient unit. The effective amount of the microorganism of the invention may be between 10³ and 10⁹ cfu per gram of rhizospheric soil. The effective amount of the supernatant of the invention may be between 1 mL and 100 L per m² of soil surface. The effective amount of the fertiliser, substrate, or active solid support may be an amount which results in between 10³ and 10⁹ cfu per gram of rhizospheric soil. Said amounts may be applied in a single administration or in several administrations.

Virtually any plant may be protected against environmental stress by this method. By way of illustration, any plant having agricultural interest may be used for this method, including plants of interest in human or animal food, plants of ornamental interest, plants of forestry interest, plants of energy interest, and so on. Non-limiting examples include oat, barley, corn, wheat, tomato, pepper, cucumber, eggplant, grass, sorghum, rose bushes, geraniums, daisies, soya, any tree, such as fruit trees, oak tree, walnut tree, beech tree, pine tree, etc.

### 3.4. Methods for solubilising insoluble compounds

### 3.4.1. Method for solubilising insoluble phosphates in a solid substrate

In another aspect, the present invention relates to a method for solubilising an insoluble phosphate in a solid substrate, hereinafter "the method for solubilising an insoluble phosphate in a solid substrate of the invention", comprising the step of applying the strain according to the invention to the solid substrate.

Alternatively, the method for solubilising an insoluble phosphate in a soil of the invention comprises the step of applying the supernatant of the invention to the soil.

It will be readily appreciated that the strain of the invention may be applied in different ways, i.e. directly to the solid substrate by injection, or as part of a fertiliser, or adhered to a substrate or solid support.

Alternatively, the method for solubilising an insoluble phosphate in a solid substrate of the invention may comprise the step of applying the fertiliser of the invention to the solid substrate.

Alternatively, the method for solubilising an insoluble phosphate in a solid substrate of the invention may comprise the step of applying the substrate of the invention to the solid substrate.

Alternatively, the method for solubilising an insoluble phosphate in a solid substrate of the invention may comprise the step of applying the active solid support of the invention to the solid substrate.

The terms "supernatant of the invention", "fertiliser of the invention", "substrate of the invention" and "active solid support of the invention" ", have been described previously in detail and their definitions and embodiments are included here by reference.

The solid substrate may contain at least one source of an insoluble phosphate. Virtually any solid substrate containing an insoluble phosphate can be treated according to this process for completely or partially reducing the insoluble phosphate content; nevertheless, in a particular embodiment, said solid substrate containing an insoluble phosphate to be treated is rock phosphate, such as the rock phosphate used to produce phosphate fertilisers with variable insoluble phosphate content.

This process can be used to solubilise all or part of the insoluble phosphate present in the solid substrate containing an insoluble phosphate, for example rock phosphate used for producing phosphate fertilisers, for the purpose of reducing the insoluble phosphate content present in said phosphate fertilisers obtained from said rock phosphate.

The solid substrate containing an insoluble phosphate to be treated may be mixed with a culture of the variant strain of the invention, or with a substrate of the invention, or with an active solid support of the invention, at a bacterial population density which may vary within a wide range. In a particular embodiment, at least 10 cfu per ml, at least 10² cfu per ml, at least 10³ cfu per ml, at least 10⁴ cfu per ml, at least 10⁵ cfu per ml, at least 10⁶ cfu per ml, at least 10⁷ cfu per ml, at least 10⁸ cfu per ml, at least 10⁹ cfu per ml, or more are inoculated.

Following inoculation, the strain of the invention is left to act for at least 1 hour, at least 2 hours, at least 3 hours, at least 4 hours, at least 5 hours, at least 10 hours, at least 15 hours, at least 20 hours, at least 24 hours, or longer. The incubation is preferably done under aerobic conditions. If desired, the strain of the invention is removed by conventional methods, such as by means of decantation, precipitation with electrolytes, centrifugation, etc. If desired, the removed microorganisms of the strain of the invention can be reused in a new method of solubilisation of an insoluble phosphate and/or insoluble iron, in a solid substrate or in a soil containing an insoluble phosphate and/or insoluble iron.

This process can be carried out in a reactor, pond or the like in which the solid substrate containing an insoluble phosphate to be treated is introduced, duly equipped with means for feeding and draining water, solid substrate, inoculation of microorganisms of the invention and recovery of the same. If necessary, the mixture (solid substrate, water and strain of the invention optionally forming part of a fertiliser, substrate, or active solid support) is supplemented with a carbon source and/or with a nitrogen source and/or essential nutrients, for the purpose of facilitating survival of the variant strain of the invention. By way of illustration, suitable amounts of micronutrient solution along with suitable amounts of magnesium, cobalt and molybdenum, typically in the micromolar order, can be added to optimize the process; nevertheless, in any case the choice and amount of nutrients and micronutrients to be added will depend on the composition of the solid substrate containing an insoluble phosphate (e.g., rock phosphate) to be treated and on the microbiological demand.

### 3.4.2. Method for solubilising insoluble phosphates in a soil

In another aspect, the present invention relates to a method for solubilising an insoluble phosphate in a soil, hereinafter "the method for solubilising an insoluble phosphate in a soil of the invention", comprising the step of applying the strain according the invention on the soil.

In another embodiment, an effective amount different strains of agricultural interest are further applied to the soil.

Alternatively, the method for solubilising an insoluble phosphate in a soil of the invention comprises the step of applying the supernatant of the invention to the soil. The person skilled in the art will appreciate that the solubilisation treatment of the soil containing insoluble phosphate may be carried out in a variety of different ways. For example, the step of applying the strain according the invention on the soil to be treated may be carried out by injecting a culture of at least one of the strains of the invention into the soil to be treated; or by adding to said soil to be treated a supplemented fertiliser of the invention, or by admixing into said soil to be treated an active solid support of the invention; or by sowing a seed of the invention or a root ball of the invention in said soil to be treated; or by admixing into said soil to be treated a soil according to the invention.

Thus, the method for solubilising an insoluble phosphate in a soil may comprise a step of applying the fertiliser of the invention to the soil.

Alternatively, the method for solubilising an insoluble phosphate in a soil may comprise a step of applying the substrate of the invention to the soil.

Alternatively, the method for solubilising an insoluble phosphate in a soil may comprise a step of applying the active solid support of the invention to the soil.

Alternatively, the method for solubilising an insoluble phosphate in a soil may comprise a step of applying the soil of the invention to the soil.

Alternatively, the method for solubilising an insoluble phosphate in a soil may comprise a step of sowing a seed of the invention or a root ball of the invention in said soil to be treated.

Alternatively, the method for solubilising an insoluble phosphate in a soil may comprise a step of sowing a root ball of the invention in said soil to be treated.

The terms "supernatant of the invention", "fertiliser of the invention", "substrate of the invention", "active solid support of the invention", "seed of the invention", and "root ball of the invention", have been described previously in detail and their definitions and embodiments are included here by reference.

The method for solubilising an insoluble phosphate in a soil may be carried out under aerobic conditions.

The particular amount of insoluble phosphate present in the soil may make it necessary to re-apply the strain of the invention, regardless of the form of presentation. Thus, the step of applying the strain according the invention on the soil may be carried out once, twice, three times, four times, five times, 6 times, 7 times, 8 times, 9 times, 10 times, or more, until partial or complete solubilisation of the insoluble phosphate is achieved.

Virtually any soil containing an insoluble phosphate, such as dibasic calcium phosphate, tribasic calcium phosphate, or mixtures of different insoluble phosphates, can be treated according to this method in order to completely or partially reduce the insoluble phosphate content present in the soil. In an embodiment, said soil is an agricultural topsoil, preferably a topsoil treated, for example, with fertilisers.

If necessary, the soil to be treated is supplemented with a carbon source and/or nitrogen source and/or essential nutrients to facilitate survival of the strain of the invention.

The injection of the microorganism of the invention is done for the purpose of achieving a high cell density in the soil to be treated, for example, of at least 10³ cfu per gram of soil to be treated, or of at least than 10⁴ cfu per gram of soil to be treated, or of at least than 10⁵ cfu per gram of soil to be treated, or of at least than 10⁶ cfu per gram of soil to be treated, or of at least than 10⁷ cfu per gram of soil to be treated.

In a particular embodiment, said method for the solubilisation of insoluble phosphate present in a soil containing an insoluble phosphate is carried out by means of adding to said soil to be treated a substrate of the invention, comprising the strain of the invention. Features of said substrate of the invention have previously been described. The amount of substrate of the invention which is added to the soil to be treated (soil containing an insoluble phosphate) may vary within a broad range. Nevertheless, in a particular embodiment the amount of said substrate of the invention which is added to the soil to be treated is equal to or greater than 0.01 kg of substrate of the invention per hectare (ha) of soil to be treated, typically equal to or greater than 0.1 kg/ha, preferably equal to or greater than 0.5 kg/ha of soil to be treated, even more preferably equal to or greater than 1 kg/ha of soil to be treated, for the purpose of facilitating the solubilisation of the insoluble phosphate.

In another particular embodiment, said method for the solubilisation of insoluble phosphate present in a soil containing an insoluble phosphate is carried out by means of adding to said soil to be treated an active solid support of the invention, comprising a variant strain of the invention. Features of said active solid support of the invention have previously been described. The amount of active solid support of the invention which is added to the soil to be treated (soil containing an insoluble phosphate) may vary within a broad range. Nevertheless, in a particular embodiment the amount of said active solid support of the invention which is added to the soil to be treated is equal to or greater than 0.01 kg of active solid support of the invention per hectare (ha) of soil to be treated, typically equal to or greater than 0.1 kg/ha, preferably equal to or greater than 0.5 kg/ha of soil to be treated, even more preferably equal to or greater than 1 kg/ha of soil to be treated, for the purpose of facilitating the solubilisation of the insoluble phosphate.

In another particular embodiment, said method for the solubilisation of insoluble phosphate present in a soil containing an insoluble phosphate is carried out by means of adding to said soil to be treated a fertiliser of the invention comprising a variant strain of the invention. The features of said fertiliser of the invention have been previously described. The amount of fertiliser of the invention which is added to the soil to be treated (soil containing an insoluble phosphate) may vary within a wide range, depending on, among other factors, the amount of insoluble phosphate present in the soil to be treated and on the amount of strain of the invention present in the fertiliser of the invention.

In another particular embodiment, the method for the solubilisation of insoluble phosphate present in a soil containing insoluble phosphate is carried out by means of sowing said soil to be treated with a seed of the invention comprising the strain of the invention. The features of said seed of the invention have been previously described. The amount of supplemented seeds of the invention which are sowed in the soil to be treated (soil containing insoluble phosphate) may vary within a wide range, depending on the density required by the farmer.

In another particular embodiment, the method for the solubilisation of insoluble phosphate present in a soil containing insoluble phosphate is carried out by means of sowing said soil to be treated with a root ball of the invention comprising the strain of the invention. The features of said root ball of the invention have been previously described. The amount of supplemented root balls of the invention which are sowed in the soil to be treated (soil containing insoluble phosphate) may vary within a wide range, depending on the density required by the farmer.

The methods of solubilisation of insoluble phosphates described above provide a number of advantages, including the following:
- high specificity in the solubilisation of insoluble phosphates;
- they work in a broad range of phosphate concentrations, typically between 0.01% and 95% (w/w) of phosphate; and
- they are highly versatile since they can be used *in situ* for solubilising said compounds in soils or in reactors for solubilising phosphate, which can be used as fertiliser *per se* or in mixtures with other compounds.

### 3.4.3. Method for solubilising iron in a solid substrate

In another aspect, the present invention relates to a method for solubilising insoluble iron in a solid substrate, hereinafter "the method for solubilising insoluble iron in a solid substrate of the invention", comprising the step of applying at least one variant strain according the invention on the solid substrate.

In an embodiment, an effective amount of BIRD-6 is applied to the solid substrate.

Alternatively, the method for solubilising insoluble iron in a solid substrate of the invention comprises the step of applying the supernatant of the invention to the solid substrate.

It will be readily appreciated that the strain of the invention may be applied in different ways, i.e. directly to the solid substrate, or as part of a fertiliser, adhered to a solid support, or as part of a soil.

Alternatively, the method for solubilising insoluble iron in a solid substrate of the invention may comprise the step of applying the fertiliser of the invention to the solid substrate.

Alternatively, the method for solubilising insoluble iron in a solid substrate of the invention may comprise the step of applying the substrate of the invention to the solid substrate.

Alternatively, the method for solubilising insoluble iron in a solid substrate of the invention may comprise the step of applying the active solid support of the invention to the solid substrate.

Alternatively, the method for solubilising insoluble iron in a solid substrate of the invention may comprise the step of applying the soil of the invention to the solid substrate.

The terms "microorganism of the invention", "supernatant of the invention", "fertiliser of the invention", "substrate of the invention", and "active solid support of the invention", have been described previously in detail and their definitions and embodiments are included here by reference.

The solid substrate may contain at least one source of insoluble iron. Virtually any solid substrate containing an insoluble iron (for example, insoluble iron of the iron oxide type or any other form of iron) can be treated according to this method in order to completely or partially reduce the insoluble iron content. Non-limiting examples of solid substrates containing an insoluble iron include phosphate rock, such as phosphate rock used for producing phosphate fertilisers with a variable insoluble phosphate content, and a soil, such as an agricultural topsoil, a topsoil treated with fertilisers, etc.

The method may optionally comprise a step of removing the microorganisms of the strain of the invention.

For carrying out this method, the solid substrate containing insoluble iron to be treated is mixed with water and the resulting mixture is put in contact with a culture of the strain of the invention, or with a fertiliser of the invention, or with a substrate of the invention, or with an active solid support of the invention, at a bacterial population density that may vary within a broad range. In a particular embodiment, at least 10³ cfu of the strain of the invention per ml of water are inoculated. After the inoculation, the strain of the invention are left to act, and at the end of 1 to 24 hours, if desired, said microorganisms are removed by conventional methods, for example, by means of decantation, precipitation with electrolytes, centrifugation, etc. If desired, the removed strain of the invention can be reused in a new method of microbiological solubilization, under aerobic conditions, of insoluble phosphate and/or insoluble iron in a solid substrate or in a soil containing insoluble phosphate and/or insoluble iron.

This method can be carried out in a reactor, or pool, in which the solid substrate containing insoluble iron to be treated is introduced, and which is duly equipped with means for the feeding in and discharging of water, solid substrate, the inoculation of the strain of the invention, and the recovery thereof. If necessary, the mixture (solid substrate, water, and strain of the invention that are optionally part of a substrate or an active solid support of the invention) is supplemented with a carbon source and/or with a nitrogen source and/or essential nutrients for the purpose of facilitating survival of the strain of the invention. By way of illustration, suitable amounts of a micronutrient solution together with suitable amounts of magnesium, cobalt and molybdenum, typically in the micromolar order, can be added to optimize the method. Nevertheless, the selection and amount of nutrients and micronutrients to be added would depend, in any case, on the composition of the solid substrate containing insoluble iron to be treated and on the microbiological demand.

This method can be used for solubilising all or part of the insoluble iron present in the solid substrate containing insoluble iron for the purpose of reducing the insoluble iron content present in said solid substrate.

### 3.4.4. Method for solubilising iron in a soil

In another aspect, the invention relates to a process for the solubilisation of insoluble iron present in a soil containing insoluble iron, hereinafter "the method for solubilising insoluble iron in a soil of the invention", comprising contacting said soil containing insoluble iron with the strain of the invention.

In another embodiment, an effective amount of different strains of agricultural interest are further applied to the soil containing insoluble iron.

Alternatively, the method for solubilising insoluble iron in a soil of the invention comprises the step of applying the supernatant of the invention to the soil containing insoluble iron.

The person skilled in the art will appreciate that the solubilisation treatment of the soil containing insoluble iron may be carried out in a variety of different ways. For example, the step of applying the strain according the invention on the soil to be treated may be carried out by injecting a culture of at least one of the strains of the invention into the soil to be treated; or by adding to said soil to be treated a supplemented fertiliser of the invention, or by admixing into said soil to be treated an active solid support of the invention; or by sowing a seed of the invention or a root ball of the invention in said soil to be treated; or by admixing into said soil to be treated a soil according to the invention.

Alternatively, the method for solubilising insoluble iron in a soil of the invention may comprise the step of applying the fertiliser of the invention to the solid substrate. Alternatively, the method for solubilising insoluble iron in a soil of the invention may comprise the step of applying the substrate of the invention to the solid substrate. Alternatively, the method for solubilising insoluble iron in a soil of the invention may comprise the step of applying the solid support of the invention to the solid substrate.

Alternatively, the method for solubilising an insoluble iron in a soil of the invention may comprise a step of sowing a root ball of the invention in said soil to be treated.

The terms "supernatant of the invention", "fertiliser of the invention", "substrate of the invention", "active solid support of the invention", "seed of the invention", and "root ball of the invention", have been described previously in detail and their definitions and embodiments are included here by reference.

The particular amount of insoluble iron present in the soil may make it necessary to re-apply the strain of the invention, regardless of the form of presentation. Thus, the step of applying the strain according the invention on the soil may be carried out once, twice, three times, four times, five times, 6 times, 7 times, 8 times, 9 times, 10 times, or more, until partial or complete solubilisation of the insoluble iron is achieved.

Virtually any soil containing insoluble iron (e.g., insoluble iron of the iron oxide type or any another form of iron) can be treated according to this process for removing all or part of the insoluble iron for completely or partially reducing the insoluble iron content present in the soil to be treated; nevertheless in a particular embodiment, said soil containing insoluble iron to be treated is a soil, such as an agricultural topsoil, a topsoil treated with fertilisers, etc. If necessary, the soil to be treated is supplemented with a carbon source and/or with a nitrogen source and/or with essential nutrients to facilitate survival of the microorganisms of the variant strain of the invention.

The injection of the microorganism of the invention is done for the purpose of achieving a high cell density in the soil to be treated, for example, of at least 10³ cfu per gram of soil to be treated, or of at least than 10⁴ cfu per gram of soil to be treated, or of at least than 10⁵ cfu per gram of soil to be treated, or of at least than 10⁶ cfu per gram of soil to be treated, or of at least than 10⁷ cfu per gram of soil to be treated.

In a particular embodiment, said method for the solubilisation of insoluble iron present in a soil containing an insoluble iron is carried out by means of adding to said soil to be treated a substrate of the invention, comprising the strain of the invention.

Features of said substrate of the invention have previously been described. The amount of substrate of the invention which is added to the soil to be treated (soil containing an insoluble iron) may vary within a broad range. Nevertheless, in a particular embodiment the amount of said substrate of the invention which is added to the soil to be treated is equal to or greater than 0.01 kg of substrate of the invention per hectare (ha) of soil to be treated, typically equal to or greater than 0.1 kg/ha, preferably equal to or greater than 0.5 kg/ha of soil to be treated, even more preferably equal to or greater than 1 kg/ha of soil to be treated, for the purpose of facilitating the solubilisation of the insoluble iron.

In a particular embodiment, said method for the solubilisation of insoluble iron present in a soil containing an insoluble iron is carried out by means of adding to said soil to be treated a supplemented soil of the invention, comprising the strain of the invention. Features of said soil of the invention have previously been described. The amount of soil of the invention which is added to the soil to be treated (soil containing an insoluble iron) may vary within a broad range. Nevertheless, in a particular embodiment the amount of said soil of the invention which is added to the soil to be treated is equal to or greater than 0.01 kg of active solid support of the invention per hectare (Ha) of soil to be treated, typically equal to or greater than 0.1 kg/Ha, preferably equal to or greater than 0.5 kg/Ha of soil to be treated, even more preferably equal to or greater than 1 kg/Ha of soil to be treated, for the purpose of facilitating the solubilisation of the insoluble iron.

In another particular embodiment, said method for the solubilisation of insoluble iron present in a soil containing an insoluble iron is carried out by means of adding to said soil to be treated a fertiliser of the invention comprising the strain of the invention. The features of said fertiliser of the invention have been previously described. The amount of fertiliser of the invention which is added to the soil to be treated (soil containing an insoluble iron) may vary within a wide range, depending on, among other factors, the amount of insoluble iron present in the soil to be treated and on the amount of strain of the invention present in the fertiliser of the invention.

In another particular embodiment, the method for the solubilisation of insoluble iron present in a soil containing insoluble iron is carried out by means of sowing said soil to be treated with a seed of the invention comprising the strain of the invention. The features of said seed of the invention have been previously described. The amount of supplemented seeds of the invention which are sowed in the soil to be treated (soil containing insoluble iron) may vary within a wide range, depending on the density required by the farmer.

In another particular embodiment, the method for the solubilisation of insoluble iron present in a soil containing insoluble iron is carried out by means of sowing said soil to be treated with a root ball of the invention comprising the strain of the invention.

The features of said root ball of the invention have been previously described. The amount of supplemented root balls of the invention which are sowed in the soil to be treated (soil containing insoluble iron) may vary within a wide range, depending on the density required by the farmer.

The solubilisation of iron, for example, by means of chelation by siderophores, prevents the iron from being available for pathogenic microorganisms, particularly plant pathogens, thus exerting biocontrol on agriculturally unwanted microbial populations. This advantage can be obtained by means of the use of the strain of the invention.

The present invention also contemplates methods for solubilising insoluble phosphates and insoluble iron in a solid substrate or a soil comprising insoluble phosphates and iron, comprising the step of applying the variant strain according to the invention to the solid substrate or soil. The skilled person will appreciate that this method is a combination of the methods for solubilising insoluble phosphates and the methods for solubilising insoluble iron that have been described previously.

### 4. Uses

In another aspect, the present invention relates to the use of the microorganism of the invention, or the supernatant of the invention, or the fertiliser, seed, root ball, substrate, or active solid support according to the invention, for stimulating plant growth.

In another aspect, the present invention relates to the use of the microorganism of the invention, or the supernatant of the invention, or the fertiliser, seed, root ball, substrate, or active solid support according to the invention, for protecting a plant against phytopathogens.

In another aspect, the present invention relates to the use of the microorganism of the invention, or the supernatant of the invention, or the fertiliser, seed, root ball, substrate, or active solid support according to the invention, for protecting a plant against environmental stress.

In another aspect, the present invention relates to the use of the microorganism of the invention, or the supernatant of the invention, or the fertiliser, seed, root ball, substrate, or active solid support according to the invention, for solubilising an insoluble phosphate in a solid substrate or field.

In another aspect, the present invention relates to the use of the microorganism of the invention, or the supernatant of the invention, or the fertiliser, seed, root ball, substrate, or active solid support according to the invention, for solubilising iron in a solid substrate or field.

In another aspect, the present invention relates to the use of the microorganism of the invention, or the supernatant of the invention, or the fertiliser, seed, root ball, substrate, or active solid support according to the invention, for solubilising an insoluble phosphate and iron in a solid substrate or field.

### 5. Methods for preparing a mineral carrier and mineral carriers containing the microorganisms of the invention

In another embodiment, the invention relates to a method for preparing a mineral carrier containing a microorganism comprising contacting a culture of the microorganism according to the invention with a mineral carrier under conditions adequate for the binding of the microorganism to the carrier.

The term "carrier", as used herein, refers to a solid support, which is a structure having a rigid or semi-rigid surface or surfaces that provides a substrate onto which microorganisms may be bound

In a preferred embodiment, the carrier is a mineral carrier. In a more preferred embodiment. The mineral carrier is a clay. In a still more preferred embodiment, the clay is sepiolite or talcum.

In another embodiment, the invention relates to a mineral carrier comprising a microorganism according to the invention.

In a preferred embodiment, the carrier is a mineral carrier. In a more preferred embodiment,. The mineral carrier is a clay. In a still more preferred embodiment, the clay is sepiolite or talcum.

### BIOLOGICAL MATERIAL DEPOSITS

The *Bacillus sp.* BIRD-6 strain strain was deposited in the Colección Española de Cultivos Tipo (in English, the Type Culture Spanish Collection, CECT) (Edificio 3 CUE, Parc Científic Universitat de Valencia, Catedrático Agustín Escardino 9, 46980 Paterna, Spain) under the conditions stipulated in the Budapest Treaty. It was deposited on 23 October 2018, and the number assigned to said deposit was CECT 9748.

These aspects of the invention exploit the use of the strain according to the invention, or the supernatant according to the invention, or the fertiliser, seed, root ball, substrate, active solid support, or soil according to the invention in the methods of the invention described previously.

### EXAMPLES

### Example 1: Isolation of the CECT 9748 Bacillus sp. BIRD-6 strain

A 10 g soil sample obtained from a garden in Pinos Genii (Granada, Spain) was suspended in M9 culture medium (Abril et al., 1989, J Bacteriology 171:6782-90), at 1:10 (w:v), the specific composition per litre of which was: 6.4 g of Na₂HPO₄ × 7H₂O, 1.5 g of KH₂PO₄, 0.25 g of NaCl, 0.5 g of NH₄Cl, 1 mL of 1 M MgSO₄, 1 ml of 6% (w:v) ferric citrate, 10 mL of 1M sodium benzoate. This initial enriched was used to inoculate the root ball of a potted lemon tree, which was placed in a greenhouse with controlled air moisture and temperature for over a month. After this time, the root and adjacent soil were harvested and placed in a sterile Erlenmeyer flask with added M9 medium and agitated in the presence of glass beads overnight. Then, serial dilutions serial dilutions were cultured in this culture medium with glucose or sodium benzoate as the carbon source, which allowed the isolation of a wide range of microorganisms.

A series of assays were run to establish which of the strains had the most properties to promote plant growth and to enhance resistance against phytopathogens. The features that were considered most important are (i) the production of compounds capable of making proteic nitrogen bioavailable, (ii) the production of compounds capable of recycling carbon sources from organic matter, (iii) the production of compounds capable of protecting the plant against phytopathogens, (iv)the production of phytohormone precursors, (v) phosphate solubilisation, and (vi) iron solubilisation.

The selection of strains having the above characteristics (i)-(vi) was as follows:
i) To test the production of compounds with digestive activity of organic nitrogen (e.g. proteins and peptides), such as enzymes to make proteic nitrogen bioavailable (e.g. proteases), the media used was solid medium containing skimmed milk (Naik et al., 2008, Microbial Ecology 56:492-504). Briefly, the specific composition per litre of this culture medium is peptone, 5 g; yeast extract, 2.5 g; glucose, 1 g; 100 mL of skimmed milk solution at 7% (w:v); agar, 15 g. Those colonies exhibiting a clear halo in the media surrounding the colony were considered positive.
ii) To test the production of compounds with organic matter digestive activity as β-glucosidases (enzymes that make sugars derived from vegetable matter bioavailable), the medium used was solid medium containing LB agar, whose specific composition per litre is: bacto-tryptone, 10 g; yeast extract, 5 g; NaCl 10 g, amended with 0.1% (w:v) esculin and 0.05% (w:v) ammonium iron (III) citrate, and 1.5% agar (Gong et al., 2012, BMC research notes 5:1). The presence of a black halo around colonies denotes a positive reaction.
iii) To test the production of hydrolytic enzymes with biocontrol activity such as amylases, lipases and proteases, several assays were performed:
   a. For amylase activity the medium used was LB agar plate (as described in (ii)) amended with 2% (w:v) soluble starch, and then, after visible growth of the microorganism, was stained with Lugol's reagent (Tindall, B. J., Sikorski, J., Smibert, R. A., & Krieg, N. R., 2007. Phenotypic characterization and the principles of comparative systematics. In Methods for General and Molecular Microbiology, Third Edition (pp. 330-93). American Society of Microbiology). The presence of a clear halo around colonies denotes a positive reaction.
   b. For lipase activity the medium used was peptone 4.3 g, casein peptone 4.3 g, NaCl 6.4 g and 1.5% agar, amended with olive oil 3% and rhodamine B 0.001% (Kouker & Jaeger, 1987, Appl Environ Microbiol. 53:211-3). After incubation, plates were placed under UV light (365 nm) and colonies surrounded with an orange fluorescent halo were considered positive.
   c. For protease activity, the assay was performed as described in (i).
iv) To test the production of plant hormones or their promoting agents, such as indoleacetic acid (IAA), the media used was amended LB (Naik *et al.,* 2008; as above), whose specific composition per litre is: bacto-tryptone, 10 g; yeast extract,
   5 g; NaCl 10 g; *L*-tryptophan, 1.025 g; sodium dodecyl sulphate (SDS) 600 mg; glycerol 10 mL; and agar 15 g. A Whatman n°1 paper disk was placed over the medium with the inoculated microorganism. After incubation, the Whatman paper disk was put into contact with Salkowsky reactive whose specific composition per litre is 2% (v/v) of a 0.5 M FeCl₃ solution in a 35% (v/v) perchloric acid solution. If a reddish colour appeared in the Whatman paper after 1 h of incubation, the production of IAA was considered positive.
v) To test inorganic and organic phosphate solubilisation, several assays were performed:
   a. For inorganic phosphate solubilisation, the medium used was Agar Pikovskaya (Naik *et al.,* 2008; as above), whose specific composition per litre is: 0.5 g (NH₄)₂SO₄; 0.2 g KCl; 0.1 g MgSO₄ × 7H₂O; 0.1 mg MnSO₄ × H₂O; 0.1 mg FeSO₄ × 7H₂O and 15 g agar. If a clear halo is observed in the medium around the colony, the production of phosphatases is considered positive.
   b. For organic phosphate solubilisation, the medium used was 0.4% (w:v) sodium phytate, 10 g D-glucose, 2 g CaCl₂, 5 g NH₄NO₃, 0.5 g KCl, 0.5 g MgSO₄ × 7H₂O, 0.01 g FeSO₄ × 7H₂O, 0.01 g MnSO₄ × H₂O and 15 g Agar (Housseinkhani et al., 2009, African J Biotechnol 8:4229-32). If a clear halo is observed in the medium around the colony, the production of phytases is considered positive.
vi) To test the production of siderophores (iron-chelating substances that allow iron assimilation by the plants), the media used was CAS Agar (Alexander & Zuberer, 1991, Biology Fertility Soils 12:39), whose specific composition per litre is 50 mL of CAS solution (Chrome Azurol S) (1.21 mg/mL); 10 mL FeCl₃ × 6H₂O (1 mM) diluted in HCI 10 mM; 40 mL hexadeciltrimetiamonium (HDTMA) (1.82 mg/mL); 30.24 g piperazine-1,4-bis (PIPES); 0.3 g KH₂PO₄; 0.5 g NaCl; 1 g NH₄Cl; 2 g glucose; 2 g mannitol; 493 mg MgSO₄ × 7H₂O; 12,5 mg CaCl₂ × 2 H₂O; 1.17 mg MnSO₄ × H₂O; 1,4 mg H₃BO₃; 0.04 mg CuSO₄ × 5H₂O; 1.2 mg ZnSO₄ × 7H₂O; 1.08 mg Na₂MoO₄ × 2 H₂O, and 3 mg casamino acids. If a yellow hallo was observed in the medium surrounding the microorganism's colony, the production of siderophores was considered positive.

The colony which produced the most plant growth-promoting and biocontrol features of all those cited before and which showed the highest efficiency, i.e., produced the most remarkable positive reaction in the shortest time in all the media, was a strain termed BIRD-6. The isolated strain exhibited bacillary form and did not display pigmentation when cultured on minimum or rich LB medium (Lennox E.S. 1955, Transduction of Linked Genetic Characters of the host by bacteriophage P1., Virology, 1:190). When cultured in minimum medium, the strain was able to use it could use sucrose, glucose, and other carbon sources and several nitrogenated compounds as a nitrogen source.

The BIRD-6 strain was confirmed to belong to the *Bacillus* species by partially sequencing the gene codifying rRNA 16S, used as a phylogenetic marker, using primers GM3 (5' -AGAGTTTGATCCTGGC- 3', SEQ ID NO: 1) and GM4 (5' - TACCTTGTTACGACTT- 3', SEQ ID NO: 2) in the presence of Taq polymerase. A DNA fragment was amplified and sequenced. The resulting sequence exhibited 99% identity to different strains of the species *Bacillus.* The sequence obtained after sequencing the PCR product using primer GM3 was as follow:
>BIRD6_16SrRNA (SEQ ID NO:3)

CATGCAAGTCGAGCGGACAGATGGGAGCTTGCTCCCTGATGTTAGCGGCGGACG
GGTGAGTAACACGTGGGTAACCTGCCTGTAAGACTGGGATAACTCCGGGAAACC
GGGGCTAATACCGGATGGTTGTTTGAACCGCATGGTTCAAACATAAAAGGTGGCT
TCGGCTACCACTTACAGATGGACCCGCGGCGCATTAGCTAGTTGGTGAGGTAAC
GGCTCACCAAGGCGACGATGCGTAGCCGACCTGAGAGGGTGATCGGCCACACTG
GGACTGAGACACGGCCCAGACTCCTACGGGAGGCAGCAGTAGGGAATCTTCCGC
AATGGACGAAAGTCTGACGGAGCAACGCCGCGTGAGTGATGAAGGTTTTCGGAT
CGTAAAGCTCTGTTGTTAGGGAAGAACAAGTACCGTTCGAATAGGGCGGTACCTT
GACGGTACCTAACCAGAAAGCCACGGCTAACTACGTGCCAGCAGCCGCGGTAAT
ACGTAGGTGGCAAGCGTTGTCCGGAATTATTGGGCGTAAAGGGCTCGCAGGCGG
TTTCTTAAGTCTGATGTGAAAGCCCCCGGCTCAACCGGGGAGGGTCATTGGAAAC
TGGGGAACTTGAGTGCAGAAGAGGAGAGTGGAATTCCACGTGTAGCGGTGAAAT
GCGTAGAGATGTGGAGGAACACCAGTGGCGAAGGCGACTCTCTGGTCTGTAACT
GACGCTGAGGAGCGAAAGCGTGGGGAGCGAACAGGATTAGATACCCTGGTAGTC
CACGCCGTAAACGATGAGTGCTAAGTGTTAGGGGGTTTCCGCCCCTTAGTGCTGC
AGCTAACGCATTAAGCACTCCGCCTGGGGAGTACGGTCGCAAGACTGAAACTCAA
AGGAATTGACGGGGGCCCGCACAAGCGGTGGAGCATGTGGTTTAATTCGAAGCA
ACACGAAGAACCTTACCAGGTCTTGACATCCTCTGACAATCCTAGAGATAGGACG
TCCCCTTCGGGGGCAGAGTGACAGGTGGTGCATGGTTGTCGTCAGCTCGTGTCG
TGAGATGTTGGGTTAAGTCCCGCAACGAGCGCAACCCTTGATCTTAGTTGCCAGC
ATTCAGTTGGGCACTCTAAGGTGACTGCCGGTGACAAACCGGAGGAAGGTGGGG
ATGACGT

The *Bacillus* sp. BIRD-6 strain was deposited in the Colección Española de Cultivos Tipo (in English, the Type Culture Spanish Collection, CECT) with accession number CECT 9748.

The ability of *Bacillus* sp. BIRD-6 (CECT 9748) to produce plant growth-promoting compounds was tested in a seed germination stimulation assay. Briefly, the assay involves sowing seeds (barley, corn, grass, Avexlll, etc.) on petri dishes containing appropriate soil mixed with bacteria of the *Bacillus* sp. BIRD-6 (CECT 9748) having a bacterial density in the order of 10⁶ a 10⁷ colony forming units (cfu) per g of soil, followed by incubation under conditions that allow seed germination (20°C and darkness). The percentage of seed germination was determined as in Examples 14-20 (please see below).

### Example 2: Hydrolysis of proteins by Bacillus sp. BIRD-6 (CECT 9748)

To test the production of compounds with organic nitrogen digestive activity and peptides, i.e. enzymes that make proteic nitrogen bioavailable, the media used was solid medium containing skimmed milk (Naik *et al.,* 2008, as above). Briefly, the specific composition per litre of this culture medium is peptone, 5 g; yeast extract, 2.5 g; glucose, 1 g; 100 mL of skimmed milk solution at 7% (w:v); agar, 15 g. BIRD-6 colonies produced a clear halo, indicating the production of proteases.

### Example 3: Degradation of organic matter by Bacillus sp. BIRD-6 (CECT 9748)

To test the production of enzymes with organic matter digestive activity as β-glucosidases, i.e. enzymes that make sugars derived from vegetable matter bioavailable, the medium used was solid medium containing LB agar, whose specific composition per litre is: bacto-tryptone, 10 g; yeast extract, 5 g; NaCl 10 g, amended with 0.1% (w:v) esculin and 0.05% (w:v) ammonium iron (III) citrate, and 1.5% agar (Gong et al., 2012; as above). BIRD-6 colonies produced a black halo, indicating the production of β-glucosidases.

### Example 4: Hydrolysis of starch into sugars by Bacillus sp. BIRD-6 (CECT 9748)

For amylase activity the medium used was LB agar plate (as described in (ii)) amended with 2% (w:v) soluble starch, and then, after visible growth of the microorganism, was stained with Lugol's reagent (Tindall et al., 2007; as above). BIRD-6 colonies produced a clear halo, indicating the degradation of starch.

### Example 5: Hydrolysis of fats by Bacillus sp. BIRD-6 (CECT 9748)

For lipase activity the medium used was peptone 4.3 g, casein peptone 4.3 g, NaCl 6.4 g and 1.5% agar, amended with olive oil 3% and rhodamine B 0.001% (Kouker & Jaeger, 1987; as above). After incubation, plates were placed under UV light (365 nm), and colonies of BIRD-6 strain showed an orange fluorescent halo, indicating the ability for degrading fats.

### Example 6: Production of phytohormones by Bacillus sp. BIRD-6 (CECT 9748)

To test the production of plant hormones or their promoting agents, such as indoleacetic acid (IAA), the media used was amended LB (Naik *et al.,* 2008; as above), whose specific composition per litre is: bacto-tryptone, 10 g; yeast extract, 5 g; NaCl 10 g; L-tryptophan, 1.025 g; sodium dodecyl sulphate (SDS) 600 mg; glycerol 10 mL; and agar 15 g. A Whatman n°1 paper disk was placed over the medium with the inoculated microorganism. After incubation, the Whatman paper disk was put into contact with Salkowsky reactive whose specific composition per litre is 2% (v/v) of a 0.5 M FeCl₃ solution in a 35% (v/v) perchloric acid solution. Colonies of the BIRD-6 strain produced a reddish colour in the Whatman paper after 1 h of incubation, indicating that the production of IAA was considered positive for this strain.

### Example 7: Solubilisation of inorganic phosphate by Bacillus sp. BIRD-6 (CECT 9748)

For inorganic phosphate solubilisation, the medium used was Agar Pikovskaya (Naik *et* al., 2008; as above), whose specific composition per litre is: 0.5 g (NH₄)₂SO₄; 0.2 g KCI; 0.1 g MgSO₄ × 7H₂O; 0.1 mg MnSO₄ × H₂O; 0.1 mg FeSO₄ × 7H₂O and 15 g agar. BIRD-6 colonies produced a clear halo, indicating the degradation of insoluble inorganic phosphate.

### Example 8: Solubilisation of organic phosphate by Bacillus sp. BIRD-6 (CECT 9748)

For organic phosphate solubilisation, the medium used was 0.4% (w:v) sodium phytate, 10 g D-glucose, 2 g CaCl₂, 5g NH₄NO₃, 0.5 g KCI, 0.5g MgSO₄ × 7H₂O, 0.01g FeSO₄ × 7H₂O, 0.01 g MnSO₄ × H₂O and 15 g Agar (Housseinkhani *et al.,* 2009; as above). BIRD-6 colonies produced a clear halo, indicating the degradation of insoluble organic phosphate.

### Example 9: Solubilisation of iron by Bacillus sp. BIRD-6 (CECT 9748)

To test the ability of solubilising iron through the production of siderophores (iron-chelating substances that allow iron assimilation by the plants), the media used was CAS Agar (Alexander & Zuberer, 1991; as above), whose specific composition per litre is 50 mL of CAS solution (Chrome Azurol S) (1.21 mg/mL); 10 mL FeCl₃ × 6H₂O (1 mM) diluted in HCI 10 mM; 40 mL hexadeciltrimetiamonium (HDTMA) (1.82 mg/mL); 30.24 g piperazine-1,4-bis (PIPES); 0.3 g KH₂PO₄; 0.5 g NaCl; 1 g NH₄Cl; 2 g glucose; 2 g mannitol; 493 mg MgSO₄ × 7H₂O; 12.5 mg CaCl₂ × 2 H₂O; 1.17 mg MnSO₄ × H₂O; 1.4 mg H₃BO₃; 0.04 mg CuSO₄ × 5H₂O; 1.2 mg ZnSO₄ × 7H₂O; 1.08 mg Na₂MoO₄ × 2 H₂O, and 3 mg casamino acids. BIRD-6 colonies produced a yellow halo, indicating the degradation of insoluble organic phosphate.

### Example 10: Use of tribasic calcium phosphate as a phosphorous source by the CECT 9748 Bacillus sp. BIRD-6 strain

A culture of the CECT 9748 *Bacillus sp.* BIRD-6 strain was inoculated with 10³ cfu/mL of microorganisms in MA modified medium (specific composition per litre: NH₄Cl, 267 mg; MgSO₄ × 7H₂O, 410 mg; KCI, 300 mg; NaCl, 200 mg; y 1 mL of an aqueous solution of iron citrate (6 g/L) and 2.5 mL of the micronutrient solution, as described by Abril *et al.,* 1989 (as above) containing a range between 0.1 and 5% (w:v) of tribasic calcium phosphate as a phosphate source and sucrose as a carbon source. The culture was incubated with shaking (between 50 y 200 rpm) in a Kuhner orbital incubator and growth was monitored through time. During the exponential growth phase, bacteria doubled every 3 to 3.5 hours and the cultures reached over 2 × 10⁹ CFU/mL after 24-48 hours of incubation.

### Example 11: Use of insoluble phosphate as a source of phosphorous by the CECT 9748 Bacillus sp. BIRD-6 strain under aerobic conditions

The procedure described in Example 10 was repeated but using molded phosphate rock containing 15 to 30% P₂O₅ whose grain size was less than 3 mm, as the phosphate source. An amount between 0.1 and 5% (w:v) phosphate rock was added observing that the duplication occurred in around 4 hours. Cultures reached cell densities between 10⁸ and 10⁹ cfu/mL. This result indicates that the CECT 9748 *Bacillus sp.* BIRD-6 strain can use insoluble phosphate as a phosphate source.

### Example 12: Solubilisation of phosphorous from phosphoric rock

Ten grams of phosphoric rock containing 27% P₂O₅ were introduced in a bucket containing 100 mL of water and was supplemented with the nutrients described in example 10 and the micronutrient solution described by Abril *et al.* (Abril *et al.,* 1989; as above) and 1% (w/v) sucrose. The solution was inoculated with a culture having at least 10³ bacteria of the CECT 9748 *Bacillus sp.* BIRD-6 strain. The culture was incubated at 30°C and aerated by shaking. Phosphorous concentration was determined every 24 hours. It was observed that between 0.1% and 0.5% of the phosphorous was solubilised after 24 h, and that the amount of solubilised phosphorous increased from 2 to 5% after 96 hours.

These results indicate that the CECT 9748 *Bacillus sp.* BIRD-6 strain can solubilise phosphate from phosphate rock.

### Example 13: Use of trace quantities of iron from phosphoric rock as an iron source by the CECT 9748 Bacillus sp. BIRD-6 strain in aerobic conditions

The CECT 9748 *Bacillus sp.* BIRD-6 strain was inoculated at 10³ cfu in M9 medium (Abril *et al.,* 1989; as above), modified with 1% (w:v) molded phosphoric rock containing 15 to 30% of P₂O₅ whose grain size was less than 3 mm, and no supplemented iron. The culture was incubated at 30°C with agitation (between 50 and 200 rpm) in a Kuhner-type orbital incubator. Viable cells were counted at different time points observing that population density doubled every 6 hours approximately, until high cell densities in the order 10⁸ to 10⁹ cfu/mL, were reached. This result indicates that the CECT 9748 *Bacillus sp.* BIRD-6 strain can use trace quantities of iron from phosphoric rock as an iron source.

### Example 14: Plant-growth promotion: Stimulation of seed germination of horticultural plants

Seeds from different horticultural plants of commercial interest in agriculture (different types of pepper, cucumber, tomato and corn) were taken and mixed with a pure culture of the CECT 9748 *Bacillus sp.* BIRD-6 strain with no agitation. At least 100 seeds of each of the selected plants treated with said microorganism were sown into Petri dishes containing 30 g of agricultural soil. Controls were carried out using the same number of seeds of each of the plants which treated with water only.

Plates were incubated in the dark at room temperature (18-22°C approximately). Three days later, germinated seeds were counted to determine the percentage of germination. The following results were obtained:
- percentage of germination of non-treated seeds: equal or less than 65%;
- percentage of germination of seeds treated with the CECT 9748 *Bacillus sp.* BIRD-6 strain: equal or over 95%.

This example illustrates that the CECT 9748 *Bacillus sp.* BIRD-6 strain stimulates the germination of seeds of horticultural plants and, therefore, promotes the growth of such plants.

### Example 15: Stimulation of the germination of barley seeds

Two hundred and fifty barley seeds were sown on Petri dishes containing 40 g of agricultural soil, with a 7.5 pH, that had been previously mixed with the CECT 9748 *Bacillus sp.* BIRD-6 strain at 10⁶ cfu/g soil. As a control, 250 barley seeds were sown on Petri dishes with the same agricultural soil (pH 7) without adding the microorganism.

The percentage of seed germination was determined after 4 days of incubation at 20°C in the dark. It was observed that the percentage of germination of barley seeds in agricultural soil without bacteria was 80%, whereas the rate of germination of seeds reached 98.6% in soil treated with *Bacillus* sp. BIRD-6 (CECT 9748).

### Example 16: Stimulation of germination of grass seeds

The assay described in Example 15 was repeated using grass seeds instead of barley. It was observed that the percentage of germination of grass seeds in soil without bacteria was 92%, whereas it reached a rate of 100% in soil supplemented with the CECT 9748 *Bacillus sp.* BIRD-6 strain. In addition, it was seen that root system of the seedlings was between 1.2x and 1.5x thicker than that of controls.

### Example 17: Stimulation of germination of corn seeds

The assay described in Example 15 was repeated using corn seeds instead of barley seeds. It was observed that the percentage of germination of corn seeds in soil without bacteria 93%, whereas it reached 100% in soil treated with the CECT 9748 *Bacillus sp.* BIRD-6 strain.

### Example 18: Promotion of plant growth: Stimulation of the growth of horticultural plants

The phytostimulating potential of the CECT 9748 *Bacillus sp.* BIRD-6 strain was tested on a soil organic substrate (peat). Briefly, a pure culture or an aqueous suspension of the CECT 9748 *Bacillus sp.* BIRD-6 strain was mixed with peat obtaining an active solid substrate (peat + microorganisms). Then this solid substrate was mixed with seeds from different horticultural plants of agricultural interest (pepper, cucumber, tomato and corn). The resulting mixtures were planted in agricultural soil and over time (depending on the plant), the percentages of seed germination, stem and root length, and times to flowering and crops were determined. As a control, the same number of seeds was planted on solid supports that were treated with water only.

The percentage of germination of the seeds treated with CECT 9748 *Bacillus sp.* BIRD-6 strain was equal or higher than 95%.

Along successive harvests it was observed that plants that had been treated with CECT 9748 *Bacillus sp.* BIRD-6 strain increased their stem length by at least 5%, and with corn, the secondary roots increased considerably up to 10% to 50% fresh weight.

As far as flowering and fruits are concerned, early crops were observed, in some cases even as soon as 10 to15 days earlier in plants treated with CECT 9748 *Bacillus sp.* BIRD-6 strain.

Again, this example demonstrates that the CECT 9748 *Bacillus sp.* BIRD-6 strain promotes stem length and increases the number of secondary roots during the first weeks of development of horticultural and herbaceous plants, and accelerates crops. It can be concluded that the CECT 9748 *Bacillus sp.* BIRD-6 strain promotes the growth of the aforementioned plants.

### Example 19: Stimulation of early growth of corn plants

To determine the phytostimulating potential of the CECT 9748 *Bacillus sp.* BIRD-6 strain on a solid support (sepiolite), a series of green-house assays were carried out in which an agricultural soil was mixed with silica sand and sepiolite to which microorganisms the CECT 9748 *Bacillus sp.* BIRD-6 strain were adhered to up to a final density of at least 10⁵ microorganisms per gram of mixture, in mixtures 2:1:1; 2:1:0,1 y 2:1:0,01 (agricultural soil: silica sand: sepiolite with *Bacillus* sp. BIRD-6). Corn seeds were sown on those well-homogenized mixtures and the percentage of germination and stem length was determined over time after sowing. Controls were carried out using untreated sepiolite.

It was observed that the stem of corn plants in crops with *Bacillus* sp. BIRD-6 were between 7% y and 10% taller than stems from corn plants sown in soils with no *Bacillus* sp. BIRD-6, although after 20 days of growth stem length of corn plants tended to be equal.

### Example 20: Supplement for seeds

Microorganisms of *Bacillus sp.* BIRD-6 (CECT 9748) were adhered to an organic polymer such as alginate and carbopol gel (a mixture of muscous mucilage (CARBOPOL 940) and triethanolamine), and the mixture was put in contact with different seeds both of agronomical and environmental interest, such as aubergine, zuchini, beans, broad beans, melon, tomato, etc., and petunia and chrysanthemum. The concentration of *Bacillus sp.* BIRD-6 (CECT 9748) was at least 10⁵ cfu per gram of seed. The seeds were sown and evaluated after a length of time appropriate for each plant type. Controls involved sowing of untreated seeds.

Satisfactory results were obtained in all cases with overtaking germination and at least a 10% increase in the size of the root and advancement of the phenological state of the plant.

### Example 21: Protection against proliferating fungal phythopathogens

Assays were done with the CECT 9748 *Bacillus sp.* BIRD-6 strain (Example 1) to determine its capacity to prevent the growth of fungi such as *Fusarium oxysporum, Rhizoctonia solanii, Colletotrichum gloeosporioides, Phithyum ultimum,* etc. A section of active mycelium of the fungi to be tested, was placed on a Petri dish in Potato Dextrose Agar, which is a specific growth medium for fungi having a specific composition per liter is 4 g potato starch; 20 g dextrose and 15 g agar (Masago et al., 1976, Phytopathology 67:425). Four drops of 20 µL of the culture of the *Bacillus sp.* BIRD-6 (CECT 9748) were placed against 20 volumes of a control microorganism that exerted no effect on the growth the of the tested fungi, e.g. *Pseudomonas putida* KT2440.

After an incubation period of no less than one week at room temperature, it was observed that the *Bacillus sp.* BIRD-6 (CECT 9748) strain inhibited the growth of fungi between 50% and 75%. This example shows the capacity of *Bacillus sp.* BIRD-6 (CECT 9748) to act as a retardant of fungi growth.

### Example 22: Effect of the culture broth of Bacillus sp. BIRD-6 (CECT 9748) on the growth of fungi

The CECT 9748 *Bacillus sp.* BIRD-6 strain was cultured in M9 minimal medium (Abril *et al*., 1989; as above), whose specific composition per liter is: 6.4 g Na₂HPO₄ × 7H₂O; 1.5 g KH₂PO₄; 0.25 g NaCl; 0.5 g NH₄Cl; 1 mL MgSO₄ 1 M; 1mL 6 ‰ (w/w) ferric citrate with 20 g/L sucrose as a carbon source. The bacterial suspension was incubated at a temperature between 15°C y 40°C with shaking until a cellular density of equal or over 10⁹ cfu/mL was reached. After that period cells were centrifuged and the culture broth was filtered through a 0.22 µm pore-size mesh. Twenty microliters of the filtered culture broth were used in an assay with *Fusarium oxysporum* and *Phityum ultimum,* as described in Example 21.

The delay in the growth of fungi lasted for over a week after the culture broth was placed in the middle of the plate. This example illustrates the capacity of the CECT 9748 *Bacillus sp.* BIRD-6 strain supernatant to act as a retarding agent against fungi proliferation.

### Example 23: Protection against proliferating bacterial phythopathogens

Assays were done with the CECT 9748 *Bacillus* sp. BIRD-6 strain to determine its capacity to prevent the growth of phytopathogenic bacteria such as *Erwinia carotovora.* The CECT 9748 *Bacillus* sp. BIRD-6 strain was cultured in M9 minimal medium (Abril *et al*., 1989; as above), whose specific composition per liter is: 6.4 g Na₂HPO₄ × 7H₂O; 1.5 g KH₂PO₄; 0.25 g NaCl; 0.5 g NH₄Cl; 1 mL MgSO₄ 1 M; 1mL 6 ‰ (w/w) ferric citrate with 20 g/L sucrose as a carbon source. The bacterial suspension was incubated at a temperature between 15°C y 40°C with shaking until a cellular density of equal or over 10⁹ cfu/mL was reached. A 20 µL drop of the culture of the CECT 9748 *Bacillus* sp. BIRD-6 strain was placed on a Petri dish in LB Agar. Once the drop was dried, a layer of LB agar 0.7% medium inoculated with 10⁵ cfu/ml of *Erwinia carotovora* was poured on the plate. After an incubation period of 24 hours at 30°C, it was observed that the *Bacillus* sp. BIRD-6 (CECT 9748) strain inhibited the growth of the bacteria observing a halo without growth of *E. carotovora* around the bacterial colony of *Bacillus* BIRD-6. This example shows the capacity of *Bacillus* sp. BIRD-6 (CECT 9748) to act as a retardant of E. *carotovora* growth.

### Example 24: Effect of the culture broth of Bacillus sp. BIRD-6 (CECT 9748) on the growth of pathogenic bacteria

The CECT 9748 *Bacillus* sp. BIRD-6 strain was cultured in M9 minimal medium (Abril et al., 1989; as above), whose specific composition per liter is: 6.4 g Na₂HPO₄ × 7 H₂O; 1.5 g KH₂PO₄; 0.25 g NaCl; 0.5 g NH₄Cl; 1 mL MgSO₄ 1 M; 1mL 6 ‰ (w/w) ferric citrate with 20 g/L sucrose as a carbon source. The bacterial suspension was incubated at a temperature between 15°C y 40°C with shaking until a cellular density of equal or over 10⁹ cfu/mL was reached. After that period cells were centrifuged and the culture broth was filtered through a 0.22 µm pore-size mesh. Ten microliters of the filtered culture broth were used in an assay with E. *carotovora,* as described in Example 23.

After incubation, it was observed that the *Bacillus* sp. BIRD-6 (CECT 9748) strain inhibited the growth of the bacteria observing a halo without growth of E. *carotovora* around the bacterial colony of *Bacillus* sp. BIRD-6. This example illustrates the capacity of the CECT 9748 *Bacillus* sp. BIRD-6 strain supernatant to act as a retarding agent against phytopathogenic bacteria proliferation.

### Example 25: Ability of CECT 9748 Bacillus sp. BIRD-6 strain to form biofilms on abiotic surfaces

The ability of CECT 9748 *Bacillus sp.* BIRD-6 strain to form biofilms on different surfaces was tested. A series of *in vitro* tests were run for which cultures were incubated for 12 h and then inoculated (1:20) in 50 mL LB or M9 minimal medium, supplemented with glucose, on polystyrene microtiter plates. After 4 h incubation at 30°C, the wells were washed with deionized water and 100 µL of a crystal violet solution at 1% (w/v) was added to each well. The plates were incubated at room temperature for 15 min. After that time the wells were washed thoroughly, and biofilm formation was counted. To that end, the blue-stained residue was solubilised by adding 200 µL ethanol twice to each well. This solution was transferred to an Eppendorf tube and 600 µL of distilled water was added. Absorbance was then measured at 600 nm in a Shimadzu UV-1700 spectrophotometer. Results showed that the microorganisms of the CECT 9748 *Bacillus sp.* BIRD-6 strain formed biofilms that reached densities of at least 10⁸ cfu/cm².

Similar assays were run to test the formation of biofilms on other surfaces such as polypropylene (Eppendorf 1.5 mL tubes), and borosilicate (glass tubes), all with positive results. The microorganism formed biofilms that reach densities of at least 10⁸ cfu/cm².

### Example 26: Ability of CECT 9748 Bacillus sp. BIRD-6 strain to form biofilms on the surface of plant roots

In order to verify the formation of biofilms by the CECT 9748 *Bacillus sp.* BIRD-6 strain on the roots of plants of agricultural interest (tomato and corn), seeds that had been previously sterilised on the surface were submerged for 30 min in a culture having 10⁸ cfu/mL of the BIRD-6 strain. After that time, seeds were washed with distilled water and placed on Petri dishes with MS medium (Murashige & Skoog, 1962, Physiologia Plantarum 15:473-97) and incubated at 25°C with light intervals of 16 hours of light and 8 hours of darkness, until a maximum length of 3 cm was reached. Then, germinating seeds were observed with a scanning electron microscope. Biofilm formation was observed in all cases.

The ten seedlings were then transplanted on seedling trays to which an organic substrate, i.e. peat, had been previously added. The growth of treated plants was observed versus that of the control untreated plants for at least 15 extra days. After this time, an increase in at least 15% to 30% in the fresh weight of the plants that had been previously inoculated with *Bacillus* sp. BIRD-6 (CECT 9748) was observed. To check for the formation of biofilms, roots were washed and the adhered cells were separated from the roots by sonification in an ultrasonic cleaner. At least 10⁹ cfu per cm² of root were recovered.

These results indicate that the formation and maintenance of the biofilm on the root formed by *Bacillus* sp. BIRD-6 (CECT 9748) occupies the ecological niche completely. This biofilm thus prevents the colonisation of other types of rhizospheric microorganisms that may turn out to be harmful for the plant's development. Moreover, it has been described that the development of biofilms on roots by plant growth-promoting microorganisms grants the plant enhanced protection against the attack of phytopathogens and against abiotic stress (high or low temperatures, draught, etc.) (Seneviratne et al., 2011, Soil Biol. Biochem 43:1059-62; Velmourougane et al., 2017, J Basic Microbiol 57:548-73; Triveni et al., 2012, Annals Microbiol. 63:1147-56; Lucas et al., 2014, Plant Physiol. Biochem. 62:44-53).

### Example 27: Plant protection from environmental stress through biofilm formation

In order to verify the ability to protect plants from environmental stresses by the CECT 9748 *Bacillus sp.* BIRD-6 strain through the formation of biofilms on the roots of plants of agricultural interest (tomato and corn), seeds that had been previously sterilised on the surface were submerged for 30 min in a culture having 10⁸ cfu/mL of the BIRD-6 strain, as indicated in example 20. After that time, seeds were washed with distilled water and placed on Petri dishes with MS medium (Murashige & Skoog, 1962, Physiologia Plantarum 15:473-97) and incubated at 25°C with light intervals of 16 hours of light and 8 hours of darkness, until a maximum length of 3 cm was reached. Parallelly, non-inoculated sterilized seeds were also germinated.

Then, non-inoculated plants were then sowed in pots containing autoclaved peat with added 100 mM NaCl to establish the control treatment, and inoculated seeds were sown in pots containing autoclaved peat with added 100 mM NaCl, in order to assess the ability of BIRD-6 to protect the plants from salinity stress.

A month after the beginning of the assay, plants were harvested and measured, BIRD-6 inoculated plants showed an increase in shoot and root length in, at least, 10% to 50%.

### Example 28: Survival of BIRD-6 in a fertilizer mixtures

A pure culture or an aqueous suspension of the CECT 9748 *Bacillus sp.* BIRD-6 strain was mixed with different formulations of liquid fertilisers, such as Herofulvat^{®}, Herocotton^{®}, Estarter, or Herovital^{®}, and similar ones supplied by the producing company Herogra. The concentration of the CECT 9748 *Bacillus sp.* BIRD-6 strain was, in all cases, at least 10⁵ cfu/mL. Once the mixtures were made, each mixture was maintained at room temperature and the viability of the microorganism was checked over time by diluting 1 mL of the mixture in water and then counting the cells after serial dilutions. After three months, all the mixtures presented the same number of viable microorganisms independently of the liquid fertilisers used in the mixture, showing a concentration of approximately 10⁸ CFU / ml. From this test it can be affirmed that the strain BIRD-6 is viable in mixutures with chemical with fertilizers.

### Example 29: Survival of BIRD-6 in a solid mixture made with an inert mineral material.

The most common formulation in agriculture for the development of solid biological products is the so-called formulation in the form of wettable powders. In order to do this, a mixture of the pure culture of the microorganism of interest is made with an inert solid support of easy bonding as the minerals belonging to the group of clays. The most commonly used clays for the manufacture of solid formulations applicable in agriculture are the so-called montmorillonite, talc, sepiolite and zeolite. This type of compounds have a high adsorption capacity so that the microorganism retention capacity is very high.

To determine the survival of *Bacillus* sp BIRD-6 in a solid mineral mixture, a pure culture of the CECT 9748 strain was mixed with three types of clays previously sterilised and in a different v:v ratio. Two proportions of volume of the microorganism culture were tested: clay v (2:10 and 3:10), using sepiolite, talc and montmorillonite as solid inert support. Once the mixtures were made, each mixture was maintained at room temperature and the viability of the microorganism was checked over time by dissolving 1 mL of the solid mixture in water and then counting the cells by serial dilution.

The inert solid montmorillonite was discarded because it couldn't be mixed properly (due to forming lumps), while the sepiolite and talc rendered homogeneous mixtures that could easily be dissolved in water. After one year, all the solid mixtures made with talc and sepiolite presented the same number of viable microorganisms independently of the proportion of culture used in the mixture, showing a concentration of approximately 10⁸ CFU / ml. From this test it can be affirmed that the strain BIRD-6 is viable in a solid mixture generated with clay after one year from its preparation.

### Demonstration of the product's safety

The following series of tests have been performed to evaluate the survival potential of the CECT 9748 *Bacillus sp.* BIRD-6 strain in the environment, as well as to determine its innocuousness for both the environment and microbes.

### Example 30: Innocuity of CECT 9748 Bacillus sp. BIRD-6 strain with respect to other plant growth-promoting microorganisms

These tests were carried by cross sowing the CECT 9748 *Bacillus sp.* BIRD-6 strain and microorganisms with plant growth-promoting potential such as *Pseudomonas putida* BIRD-1 and *Pseudomonas fluorescens* BIRD-5. All strains grow in LB medium. Briefly, microorganisms of one strain were sown on a line using a lancet; that line was cut perpendicularly by sowing with microorganisms of another strain. If the microorganisms are innocuous between them, growth along the sowing lines is homogenous. If, however, one of them exerts a detrimental effect on the rest, then an inhibition area is observed.

Results revealed that CECT 9748 *Bacillus sp.* BIRD-6 strain is innocuous for BIRD-1 and BIRD-5. The same applies to the strains BIRD-1 and BIRD-5 between them or with respect to BIRD-6. Therefore, it can be concluded that the analysed strains are not antagonistic.

### Example 31: Innocuity of CECT 9748 Bacillus sp. BIRD-6 strain with respect to other heterotrophic soil microorganisms

Corn was sown on fluvisol that had been supplemented with 10⁶ cfu of CECT 9748 *Bacillus sp.* BIRD-6 strain per gram of soil. Controls did not contain the CECT 9748 *Bacillus sp.* BIRD-6 strain. Seeds were incubated at room temperature with a light-darkness interval of 8 h of light and 8 h of darkness. The number of heterotrophic microorganisms in the root system was determined by counting the number of microorganisms that:
1) Used nitrate as an N source. To this end a modified M9 medium, as described above, was used in which NH₄Cl was replaced by 10 mM KNO₃.
2) The number of microorganisms that used benzoate as C source in the rhizosphere. To this end M9 minimal medium was used and 10 mM sodium benzoate was used as the sole carbon source.

Counts taken after 15 days revealed that the number of nitrate users was around 10⁷ cfu/g soil both in the rhizosphere of plants growing in treated soil and in the control plants. The number of microorganisms that used benzoate reached values close to 10⁸ cfu per gram of soil both in soils inoculated with *Bacillus* sp. BIRD-6 and in non-inoculated soils.

From these assays it can be concluded that CECT 9748 *Bacillus sp.* BIRD-6 strain does not exert a negative effect on the survival of natural soil microorganisms.

## Claims

1. A microorganism of the *Bacillus sp.* BIRD-6 strain with accession number CECT 9748.

2. A supernatant of a culture of the microorganism according to claim 1.

3. A fertiliser, seed, root ball, substrate or active solid support, comprising at least one microorganism according to claim 1, or a supernatant according to claim 2.

4. Method for stimulating plant growth, comprising a step of applying an effective amount of at least one microorganism according to claim 1, or a supernatant according to claim 2, or a fertiliser, substrate or active solid support according to claim 3, to the plant or, alternatively, a step of planting a seed or a root ball according to claim 3.

5. The method according to claim 4 wherein the stimulation of plant growth is caused by a stimulation of seed germination or by a stimulation of early growth.

6. Method for protecting a plant against phytopathogens, comprising a step of applying an effective amount of a microorganism according to claim 1, or a supernatant according to claim 2, or a fertiliser, substrate, or active solid support according to claim 3, to the plant or, alternatively, a step of planting a seed or a root ball according to claim 3.

7. The method according to claim 6 wherein the phytopathogen is a fungi and/or a bacteria.

8. Method for protecting a plant against environmental stress, comprising the step of applying an effective amount of at least a microorganism according to claim 1, or a supernatant according to claim 2, or a fertiliser, substrate, or active solid support according to claim 3, to the plant or, alternatively, the step of planting a seed or a root ball according to claim 3.

9. Method for solubilising an insoluble phosphate and/or insoluble iron in a solid substrate or soil, comprising the step of applying at least one microorganism according to claim 1, or a supernatant according to claim 2, or a fertiliser, substrate, or active solid support according to claim 3, to the solid substrate or field or, alternatively, the step of planting a seed or a root ball according to claim3 on the solid substrate or soil.

10. Method for preparing a mineral carrier containing a microorganism comprising contacting a culture of the microorganism according to claim 1 with a mineral carrier under conditions adequate for the binding of the microorganism to the carrier.

11. A mineral carrier comprising a microorganism according to claim 1.

12. The method according to claim 10 or the mineral carrier according to claim 11 wherein the mineral carrier is a clay.

13. The method according to claim 12 wherein the clay is sepiolite or talcum.

## Patentansprüche

1. Mikroorganismus vom Stamm *Bacillus sp.* BIRD-6 mit der Hinterlegungsnummer CECT 9748.

2. Überstand einer Kultur des Mikroorganismus nach Anspruch 1.

3. Dünger, Saatgut, Wurzelballen, Substrat oder aktiver fester Träger, aufweisend mindestens einen Mikroorganismus nach Anspruch 1, oder einen Überstand nach Anspruch 2.

4. Verfahren zum Stimulieren von Pflanzenwachstum, aufweisend einen Schritt des Applizierens einer wirksamen Menge von mindestens einem Mikroorganismus nach Anspruch 1, oder einem Überstand nach Anspruch 2, oder einem Dünger, einem Substrat oder einem aktiven festen Träger nach Anspruch 3 auf die Pflanze, oder, alternativ, einen Schritt des Pflanzens eines Saatguts oder eines Wurzelballens nach Anspruch 3.

5. Verfahren nach Anspruch 4, wobei die Stimulation des Pflanzenwachstums durch eine Stimulation von Saatgutkeimung oder durch eine Stimulation von frühem Wachstum bewirkt wird.

6. Verfahren zum Schützen einer Pflanze gegen Phytopathogene, aufweisend einen Schritt des Applizierens einer wirksamen Menge von einem Mikroorganismus nach Anspruch 1, oder einem Überstand nach Anspruch 2, oder einem Dünger, einem Substrat oder einem aktiven festen Träger nach Anspruch 3 auf die Pflanze, oder, alternativ, einen Schritt des Pflanzens eines Saatguts oder eines Wurzelballens nach Anspruch 3.

7. Verfahren nach Anspruch 6, wobei das Phytopathogen ein Pilz und/oder ein Bakterium ist.

8. Methode zum Schützen einer Pflanze gegen Umweltstress, aufweisend den Schritt des Applizierens einer wirksamen Menge von mindestens einem Mikroorganismus nach Anspruch 1, oder einem Überstand nach Anspruch 2, oder einem Dünger, einem Substrat oder einem aktiven festen Träger nach Anspruch 3 auf die Pflanze, oder, alternativ, den Schritt des Pflanzens eines Saatguts oder eines Wurzelballens nach Anspruch 3.

9. Verfahren zur Solubilisierung eines unlöslichen Phosphats und/oder unlöslichen Eisens in einem festen Substrat oder Boden, aufweisend den Schritt des Applizierens von mindestens einem Mikroorganismus nach Anspruch 1, oder einem Überstand nach Anspruch 2, oder einem Dünger, einem Substrat oder einem aktiven festen Träger nach Anspruch 3 auf das feste Substrat oder Feld, oder, alternativ, den Schritt des Pflanzens eines Saatguts oder eines Wurzelballens nach Anspruch 3 in das feste Substrat oder den Boden.

10. Verfahren zum Herstellen eines mineralischen Trägers enthaltend einen Mikroorganismus, wobei das Verfahren das Kontaktieren einer Kultur des Mikroorganismus nach Anspruch 1 mit einem mineralischen Träger unter Bedingungen, die adäquat für das Binden des Mikroorganismus an den Träger sind, aufweist.

11. Mineralischer Träger aufweisend einen Mikroorganismus nach Anspruch 1.

12. Verfahren nach Anspruch 10 oder mineralischer Träger nach Anspruch 11, wobei der mineralische Träger ein Ton ist.

13. Verfahren nach Anspruch 12, wobei der Ton Sepiolith oder Talkum ist.

## Revendications

1. Un microorganisme de *Bacillus sp.* souche BIRD-6 avec numéro d'accession CECT 9748.

2. Un surnageant d'une culture du microorganisme selon la revendication 1.

3. Un engrais, une semence, une motte pour racines, un substrat ou un support solide actif, comprenant au moins un microorganisme selon la revendication 1, ou un surnageant selon la revendication 2.

4. Procédé de stimulation de la croissance des plantes, comprenant une étape consistant à appliquer une quantité efficace d'au moins un microorganisme selon la revendication 1, ou d'un surnageant selon la revendication 2, ou d'un engrais, substrat ou support solide actif selon la revendication 3, à la plante ou, alternativement, une étape consistant à planter une graine ou une motte pour racines selon la revendication 3.

5. Le procédé selon la revendication 4 dans lequel la stimulation de la croissance des plantes est provoquée par une stimulation de la germination de graines ou par une stimulation de la croissance précoce.

6. Procédé de protection d'une plante contre des phytopathogènes, comprenant une étape consistant à appliquer une quantité efficace d'un microorganisme selon la revendication 1, ou d'un surnageant selon la revendication 2, ou d'un engrais, substrat, ou support solide actif selon la revendication 3, à la plante ou, alternativement, une étape consistant à planter une graine ou une motte pour racines selon la revendication 3.

7. Le procédé selon la revendication 6 dans lequel le phytopathogène est un champignon et/ou une bactérie.

8. Procédé de protection d'une plante contre les stress environnementaux, comprenant l'étape consistant à appliquer une quantité efficace d'au moins un microorganisme selon la revendication 1, ou un surnageant selon la revendication 2, ou un engrais, substrat, ou support solide actif selon la revendication 3, à la plante ou, alternativement, l'étape consistant à planter une graine ou une motte pour racines selon la revendication 3.

9. Procédé de solubilisation d'un phosphate insoluble et/ou de fer insoluble dans un substrat solide ou un sol, comprenant l'étape consistant à appliquer au moins un microorganisme selon la revendication 1, ou un surnageant selon la revendication 2, ou un engrais, substrat, ou support solide actif selon la revendication 3, au substrat solide ou au champ ou, alternativement, l'étape consistant à planter une graine ou une motte pour racines selon la revendication 3 sur le substrat solide ou le sol.

10. Procédé de préparation d'un support minéral contenant un microorganisme comprenant le fait de mettre en contact une culture du microorganisme selon la revendication 1 avec un support minéral dans des conditions adéquates pour la liaison du microorganisme au support.

11. Un support minéral comprenant un microorganisme selon la revendication 1.

12. Le procédé selon la revendication 10 ou le support minéral selon la revendication 11, le support minéral étant une argile.

13. Le procédé selon la revendication 12, dans lequel l'argile est de la sépiolite ou du talc.
